# EUROPÄISCHE PATENTSCHRIFT

(11) **EP 2 627 730 B1**
(45) Veröffentlichungstag und Bekanntmachung des Hinweises auf die Patenterteilung: **06.05.2020**
(21) Anmeldenummer: 11761008.9
(22) Anmeldetag: 15.09.2011
(51) Int. Cl.: C09K 11/06, H05B 33/10, H05B 33/14, C07D 209/82

(54) **FORMULIERUNGEN FÜR ORGANISCHE ELEKTROLUMINESZENZVORRICHTUNGEN**
FORMULATIONS FOR ORGANIC ELECTROLUMINESCENCE DEVICES
FORMULATIONS POUR DISPOSITIFS ÉLECTROLUMINESCENTS ORGANIQUES

(30) Priorität: 14.10.2010 DE 102010048497
(43) Veröffentlichungstag der Anmeldung: 21.08.2013
(73) Patentinhaber: Merck Patent GmbH, 64293 Darmstadt (DE)
(72) Erfinder: LUDEMANN, Aurélie, 60322 Frankfurt am Main (DE); ANEMIAN, Rémi, Manouk, Seoul 657-169 (KR); HEUN, Susanne, 65812 Bad Soden (DE); HEIL, Holger, 60389 Frankfurt am Main (DE); EBERLE, Thomas, 76829 Landau (DE)
(86) Internationale Anmeldenummer: PCT/EP2011/004645
(87) Internationale Veröffentlichungsnummer: WO 2012/048779

(56) Entgegenhaltungen:
- EP-A1- 2 141 152
- WO-A1-2008/086851
- US-A1- 2009 302 752
- INOMATA H ET AL: "High efficiency organic electrophosphorescent diodes using 1,3,5-triazine electron transport materials", CHEMISTRY OF MATERIALS, AMERICAN CHEMICAL SOCIETY, WASHINGTON, US, Bd. 16, Nr. 7, 6. April 2004 (2004-04-06), Seiten 1285-1291, XP002508000, ISSN: 0897-4756, DOI: 10.1021/CM034689T [gefunden am 2004-03-03]
- KYUNG SOO SON ET AL: "Analyzing Bipolar Carrier Transport Characteristics of Diarylamino-Substituted Heterocyclic Compounds in Organic Light-Emitting Diodes by Probing Electroluminescence Spectra", CHEMISTRY OF MATERIALS, Bd. 20, Nr. 13, 1. Juli 2008 (2008-07-01), Seiten 4439-4446, XP55013230, ISSN: 0897-4756, DOI: 10.1021/cm8004985
- DE SILVA C R ET AL: "Europium beta-diketonates for red-emitting electroluminescent devices", THIN SOLID FILMS, ELSEVIER-SEQUOIA S.A. LAUSANNE, CH, Bd. 517, Nr. 2, 28. November 2008 (2008-11-28), Seiten 957-962, XP025625030, ISSN: 0040-6090, DOI: 10.1016/J.TSF.2008.08.118 [gefunden am 2008-08-23]

## Beschreibung

Die vorliegende Erfindung betrifft Formulierungen, insbesondere zur Verwendung in organischen Elektrolumineszenzvorrichtungen, umfassend eine Carbazolverbindung, eine Elektronentransportverbindung, eine Triplett-Emitter-Verbindung und mindestens ein Lösungsmittel, wobei die Elektronentransportverbindung eine Triazin-verbindung umfasst und wobei die Carbazolverbindung mindestens zwei Carbazolgruppen aufweist, die über ihre N-Atome miteinander verbunden sind. Die Erfindung ist ferner auf organische Elektrolumineszenzvorrichtungen gerichtet, welche die erfindungsgemäßen Mischungen enthalten.

Der Aufbau organischer Elektrolumineszenzvorrichtungen (OLEDs), in denen organische Halbleiter als funktionelle Materialien eingesetzt werden, ist beispielsweise in der US 4539507, der US 5151629, der EP 0676461 und der WO 98/27136 beschrieben. Eine Entwicklung im Bereich der organischen Elektrolumineszenzvorrichtungen sind phosphoreszierende OLEDs. Diese weisen aufgrund der höheren erreichbaren Effizienz im Vergleich zu fluoreszierenden OLEDs deutliche Vorteile auf.

Allerdings gibt es bei den phosphoreszierenden OLEDs noch Verbesserungsbedarf. Dies gilt insbesondere für die Effizienz und die Lebensdauer der Vorrichtungen.

Gemäß dem Stand der Technik werden elektronenleitende Materialien, unter anderem Ketone (z.B. gemäß der WO 04/093207) oder Triazinderivate (z.B. gemäß der DE 102008036982) als Matrixmaterialien für phosphoreszierende Emitter verwendet. Insbesondere mit Ketonen werden niedrige Betriebsspannungen und lange Lebensdauern erzielt, was diese Verbindungsklasse zu einem sehr interessanten Matrixmaterial macht. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtungen.

Aus dem Stand der Technik sind weiterhin organische Elektrolumineszenzvorrichtungen bekannt, welche einen phosphoreszierenden Emitter dotiert in einer Mischung aus zwei Matrixmaterialien enthalten.

In der US 2007/0252516 werden phosphoreszierende organische Elektrolumineszenzvorrichtungen offenbart, welche eine Mischung aus einem loch- und einem elektronenleitenden Matrixmaterial aufweisen. Für diese OLEDs wird eine verbesserte Effizienz offenbart. Ein Einfluss auf die Lebensdauer ist nicht erkennbar.

In der US 2007/0099026 werden weiß emittierende organische Elektrolumineszenzvorrichtungen offenbart, wobei die grün bzw. rot emittierende Schicht einen phosphoreszierenden Emitter und eine Mischung aus einem loch- und einem elektronenleitenden Matrixmaterial aufweist. Als lochleitende Materialien werden unter anderem Triarylamin- und Carbazolderivate offenbart. Als elektronenleitende Materialien werden unter anderem Aluminium- und Zinkverbindungen, Oxadiazolverbindungen und Triazin- bzw. Triazolverbindungen offenbart. Auch für diese OLEDs sind noch weitere Verbesserungen wünschenswert.

In der WO 2008/127063 A2 wird eine Elektrolumineszenzvorrichtung offenbart, welche eine Kathode, eine Anode und dazwischen eine lichtemittierende Schicht aufweist, welche eine Triplett-Emitterverbindung in einem Host enthält. Der Host ist dabei eine Mischung aus einer elektronentransportierenden und einer lochtransportierenden Co-Host-Einheit. Diese Vorrichtung benötigt jedoch zwingend eine Elektroneninjektionsschicht.

In der US 2009/0302752 A1 wird eine Elektrolumineszenzvorrichtung offenbart, welche eine Kathode, eine Anode und dazwischen eine lichtemittierende Schicht aufweist, welche eine Triplett-Emitterverbindung in einem Host enthält. Der Host kann dabei eine Mischung aus einer elektronentransportierenden Ketonverbindung und einer lochtransportierenden Carbazolverbindung sein.

Meist werden diese Mischungen im Hochvakuum aufgedampft, was einerseits sehr aufwändig ist und andererseits bei den hohen Temperaturen, die für den Aufdampfprozess notwendig sind, bereits zur Zersetzung einer Vielzahl von Verbindungen führt. Dies hat einen negativen Einfluss auf eine so hergestellte Elektrolumineszenzvorrichtung.

Die der Erfindung zugrunde liegende technische Aufgabe war deshalb die Bereitstellung einer Formulierung, welche sich einfach verarbeiten lässt und in einer organischen Elektrolumineszenzvorrichtung zu einer sehr hohen Lebensdauer und guten Effizienz führt.

Diese Aufgabe wird erfindungsgemäß gelöst durch eine Formulierung enthaltend eine Carbazolverbindung, eine Elektronentransportverbindung, eine Triplett-Emitter-Verbindung und mindestens ein Lösungsmittel, wobei die Elektronentransportverbindung eine Triazinverbindung umfasst und wobei die Carbazolverbindung mindestens zwei Carbazolgruppen aufweist, die über ihre N-Atome miteinander verbunden sind.

Neben dem mindestens einen Lösungsmittel enthält die erfindungsgemäße Formulierung:
- mindestens eine Carbazolverbindung,
- mindestens eine Elektronentransportverbindung und
- mindestens eine Triplett-Emitter-Verbindung.

Der Anteil der Carbazolverbindung in der erfindungsgemäßen Formulierung liegt dabei vorzugsweise im Bereich von 10 bis 70 Gew.-%, besonders bevorzugt im Bereich von 20 bis 60 Gew.-% und ganz besonders bevorzugt im Bereich von 30 bis 50 Gew.-%, bezogen auf die Mischung aus Carbazol-, Elektronentransport- und Triplett-Emitter-Verbindung.

Der Anteil der Elektronentransportverbindung, und damit der Anteil Triazinverbindung, in der erfindungsgemäßen Formulierung liegt vorzugsweise im Bereich von 10 bis 70 Gew.-%, besonders bevorzugt im Bereich von 20 bis 60 Gew.-% und ganz besonders bevorzugt im Bereich von 30 bis 50 Gew.-%, bezogen auf die Mischung aus Carbazol-, Elektronentransport- und Triplett-Emitter-Verbindung.

Das Gewichtsverhältnis zwischen der Carbazolverbindung und der Elektronentransportverbindung liegt vorzugsweise zwischen 10:1 und 1:10, besonders bevorzugt zwischen 7:1 und 1:7, und ganz besonders bevorzugt zwischen 4:1 und 1:4.

Der Anteil der Triplett-Emitter-Verbindung in der erfindungsgemäßen Formulierung liegt dabei vorzugsweise im Bereich von 0,01 bis 50 Gew.-%, besonders bevorzugt im Bereich von 0,1 bis 40 Gew.-% und ganz besonders bevorzugt im Bereich von 1 bis 30 Gew.-%, bezogen auf die Mischung aus Carbazol-, Elektronentransport- und Triplett-Emitter-Verbindung.

Die Konzentration der Mischung aus Carbazol-, Elektronentransport- und Triplett-Emitter-Verbindung in der erfindungsgemäßen Formulierung liegt vorzugsweise im Bereich von 1 bis 100 g/l, besonders bevorzugt im Bereich von 5 bis 50 g/l uns ganz besonders bevorzugt im Bereich von 10 bis 30 g/l.

Vorzugsweise sind die N-Atome der Carbazolgruppen über ein aromatisches oder heteroaromatisches Ringsystem miteinander verbunden.

In einer Ausführungsform der vorliegenden Erfindung ist die Carbazolverbindung vorzugsweise eine Verbindung der Formel (1) 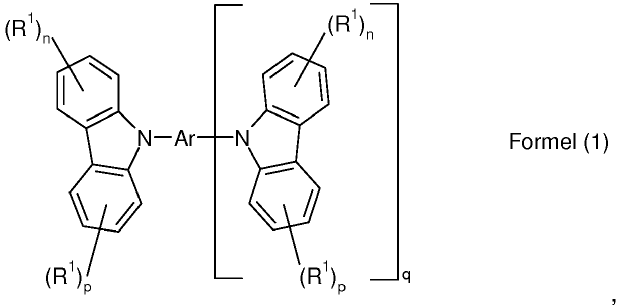 wobei für die verwendeten Symbole und Indices gilt:
- Ar: ist bei jedem Auftreten ein aromatisches oder heteroaromatisches Ringsystem mit 5 bis 60 aromatischen Ringatomen, welches mit einem oder mehreren Resten R substituiert sein kann;
- R, R¹: ist bei jedem Auftreten gleich oder verschieden H, D, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, -CR²=CR²(Ar¹), OSO₂R², eine geradkettige Alkyl-, Alkoxy oder Thioalkoxygruppe mit 1 bis 40 C-Atomen oder eine verzweigte oder cyclische Alkyl-, Alkoxy oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R² substituiert sein kann, oder eine Aryloxy- oder Heteroaryloxygruppe mit 5 bis 60 aromatischen Ringatomen, die mit einem oder mehreren Resten R² substituiert sein kann, oder eine Kombination dieser Systeme; dabei können zwei oder mehrere Substituenten R, R¹ bzw. R und R¹ auch miteinander ein mono- oder polycyclisches, aliphatisches, aromatisches oder heteroaromatisches Ringsystem bilden;
- Ar¹: ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
- R²: ist bei jedem Auftreten gleich oder verschieden H oder ein aliphatischer, aromatischer und/oder heteroaromatischer Kohlenwasserstoffrest mit 1 bis 20 C-Atomen; oder ein aromatisches oder heteroaromatisches Ringsystem mit 5 bis 60 aromatischen Ringatomen, welches mit einem oder mehreren Resten R substituiert sein kann; dabei können zwei oder mehrere Substituenten R² auch miteinander ein mono oder polycyclisches, aliphatisches, aromatisches oder heteroaromatisches Ringsystem bilden;
- n: ist bei jedem Auftreten gleich oder verschieden 0, 1 , 2, 3 oder 4;
- p: ist bei jedem Auftreten gleich oder verschieden 0, 1 , 2, 3 oder 4;
- q: ist 1, 2, 3, 4 oder 5.

Wenn der Index q gleich 1 ist, bedeutet dies, dass Ar eine bivalente Gruppe darstellt. Wenn der Index q größer als 1 ist, bedeutet dies, dass insgesamt drei oder mehr Carbazolgruppen an das aromatische Ringsystem Ar gebunden sind. Ar ist für q = 2 eine trivalente Gruppe und für q > 2 eine entsprechend höhervalente Gruppe. Bevorzugt ist der Index q = 1 oder 2, besonders bevorzugt ist q = 1.

Bevorzugt weisen die erfindungsgemäß eingesetzten Carbazolverbindungen eine Glasübergangstemperatur T_{g} von mehr als 120°C auf, besonders bevorzugt von mehr als 140°C.

Die Carbazolgruppe dient in der vorliegenden Erfindung vornehmlich als Matrixmaterial und/oder als Lochtransportmaterial. Ein lochtransportierendes Material ist in der vorliegenden Anmeldung gekennzeichnet durch ein HOMO von vorzugsweise mehr als -5,4 eV. Ein elektronentransportierendes Material ist in der vorliegenden Anmeldung gekennzeichnet durch ein LUMO von vorzugsweise weniger als -2,4 eV. Dabei erfolgt die Bestimmung der HOMO- und LUMO-Lagen und der Energielücke so, wie im Beispielteil ausführlich beschrieben.

Eine Arylgruppe gemäß der vorliegenden Erfindung enthält 6 bis 60 C-Atome; eine Heteroarylgruppe gemäß der vorliegenden Erfindung enthält 2 bis 60 C-Atome und mindestens ein Heteroatom, mit der Maßgabe, dass die Summe aus C-Atomen und Heteroatomen mindestens 5 ergibt. Die Heteroatome sind vorzugsweise ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem gemäß der vorliegenden Erfindung enthält 6 bis 40 C-Atome im Ringsystem. Ein heteroaromatisches Ringsystem gemäß der vorliegenden Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind vorzugsweise ausgewählt aus N, O und/oder S. Unter einem aromatischen oder heteroaromatischen Ringsystem gemäß der vorliegenden Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (vorzugsweise weniger als 10 % der von H verschiedenen Atome), wie z.B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme gemäß der vorliegenden Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Das aromatische Ringsystem enthält vorzugsweise keine Metallatome.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, vorzugsweise die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl und Octinyl verstanden.

Unter einer C₁- bis C₄₀-Alkoxygruppe werden vorzugsweise Methoxy, Trifluormethoxy, Ethoxy, n-Propoxy, i-Propoxy, n-Butoxy, i-Butoxy, s-Butoxy, t-Butoxy und 2-Methylbutoxy verstanden.

Unter einem aromatischen oder heteroaromatischen Ringsystem mit 5 bis 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Erfindung sind die Indices n in der Verbindung der Formel (1) bei jedem Auftreten gleich oder verschieden 0 oder 1. Besonders bevorzugt sind die Indices n = 0.

In einer Ausführungsform ist in der Verbindung der Formel (1) der Index p gleich oder verschieden bei jedem Auftreten 0, 1 oder 2, besonders bevorzugt 0 oder 1. Wenn der Index p gleich 1 ist, ist der Substituent R¹ vorzugsweise in der 6-, 7- oder 8-Position des Carbazols gebunden und besonders bevorzugt in der 6- oder 7-Position.

Der Übersichtlichkeit halber ist in der folgenden Formel die Nummerierung der Positionen des Carbazols dargestellt:

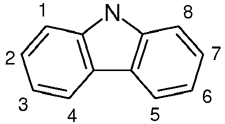

Bevorzugte Gruppen Ar und R¹ in Formel (1) enthalten nur Phenyl- und/oder Naphthylgruppen oder heteroaromatische Gruppen mit nicht mehr als zwei kondensierten aromatischen bzw. heteroaromatischen Ringen, jedoch keine größeren kondensierten aromatischen Systeme. Daher sind bevorzugte Gruppen Ar und R¹ aromatische Ringsysteme, welche aufgebaut sind aus Phenyl- und/oder Naphthylgruppen oder Verknüpfungen dieser Systeme, wie beispielsweise Biphenyl, Fluoren, Spirobifluoren, etc.

Besonders bevorzugte Gruppen Ar sind ausgewählt aus der Gruppe bestehend aus 1,2-Phenylen, 1,3-Phenylen, 1,4-Phenylen, 1,3,5-Benzol, 3,3'-Biphenyl, 4,4'-Biphenyl, 1,3,5-Triphenylbenzol, Triphenylamin, 2,7-Fluorenylen, welches durch einen oder mehrere Reste R¹ substituiert sein kann, 2,7-Spirobifluorenylen, welches durch einen oder mehrere Reste R¹ substituiert sein kann, Indenofluorenylen, welches durch einen oder mehrere Reste R¹ substituiert sein kann, 4,4'''-(1,1':2',1",2",1'''-Quarterphenyl), 4,4'-(2,2'-Dimethylbiphenyl), 4,4'-(1,1'-Binaphthyl), 4,4'-Stilbenyl und Dihydrophenanthrenyl, welches durch einen oder mehrere Reste R¹ substituiert sein kann.

Besonders bevorzugte Gruppen R¹ der Carbazolverbindung sind gleich oder verschieden ausgewählt aus der Gruppe bestehend aus Phenyl, 1-Naphthyl, 2-Naphthyl, 2-Carbazolyl, 3-Carbazolyl, 9-Carbazolyl, Triphenylamin, Naphthyldiphenylamin und Dinaphthylphenylamin, welche jeweils durch einen oder mehrere Reste R substituiert sein können. Dabei können die beiden letztgenannten Gruppen über das Naphthalin in 1- oder 2-Position oder über die Phenylgruppe gebunden sein. Eine 2- bzw. 3-Carbazolylgruppe ist dabei vorzugsweise am Stickstoff durch einen aromatischen Rest R substituiert.

Bevorzugt sind weiterhin Verbindungen gemäß Formel (1) in denen das Symbol R gleich oder verschieden bei jedem Auftreten für H, N(Ar²)₂, eine geradkettige Alkylgruppe mit 1 bis 5 C-Atomen oder verzweigte Alkylgruppe mit 3 bis 5 C-Atomen, wobei jeweils eine oder mehrere nicht benachbarte CH₂-Gruppen durch -R²C=CR²- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei dieser Systeme steht. Besonders bevorzugte Reste R sind gleich oder verschieden bei jedem Auftreten H, Methyl, Ethyl, iso-Propyl, tert-Butyl, wobei jeweils ein oder mehrere H-Atome durch F ersetzt sein können, oder eine Phenyl-, Naphthyl- oder Spirobifluorenylgruppe, die jeweils mit einem oder mehreren Resten R substituiert sein kann, oder eine Kombination aus zwei dieser Systeme. Bei Verbindungen, die aus Lösung verarbeitet werden, sind auch lineare oder verzweigte Alkylketten mit bis zu 10 C-Atomen bevorzugt. Brom, Boronsäure bzw. Boronsäurederivate als Substituenten sind vor allem für die Verwendung dieser Verbindungen als Zwischenverbindungen zur Herstellung weiterer erfindungsgemäßer Verbindungen, beispielsweise Polymere, Oligomere oder Dendrimere, bevorzugt.

Bevorzugt sind weiterhin Verbindungen gemäß Formel (1) in denen das Symbol R¹ gleich oder verschieden bei jedem Auftreten entsprechend dem bevorzugten Substituenten R definiert ist oder für Ar oder F steht.

Beispiele für besonders bevorzugte Verbindungen gemäß Formel (1) sind die im Folgenden abgebildeten Verbindungen (1-1) bis (1-91).

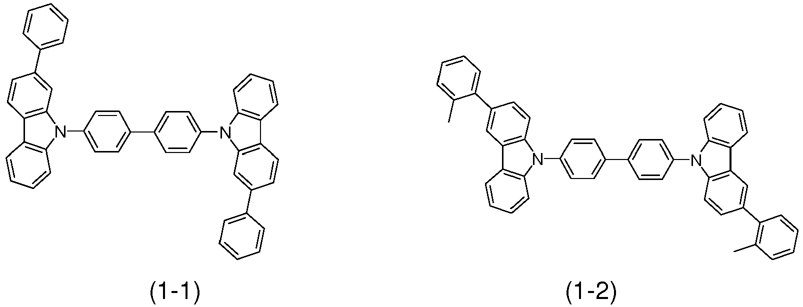

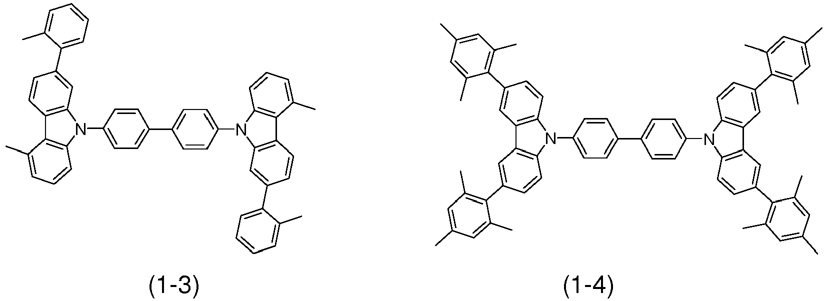

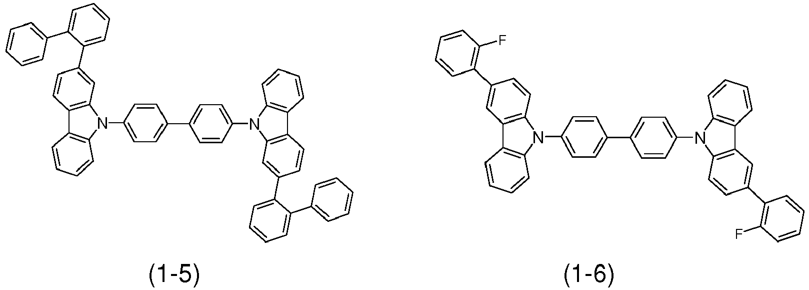

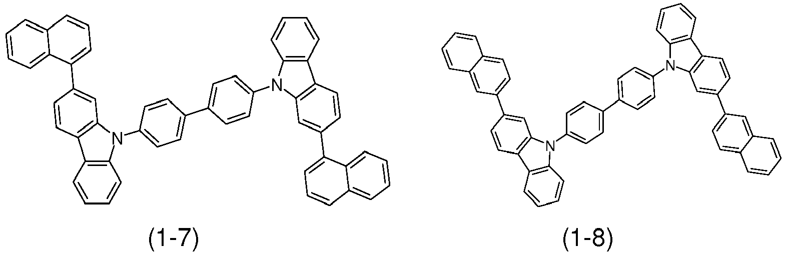

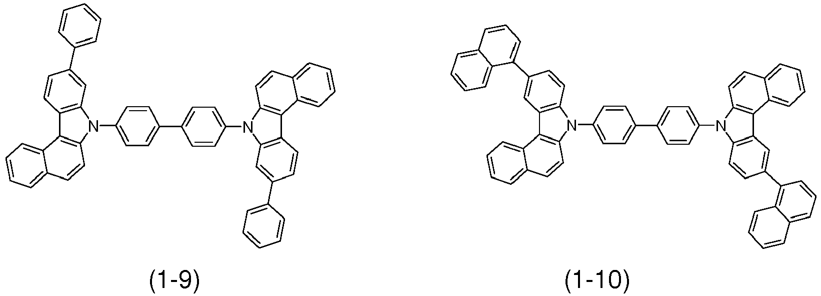

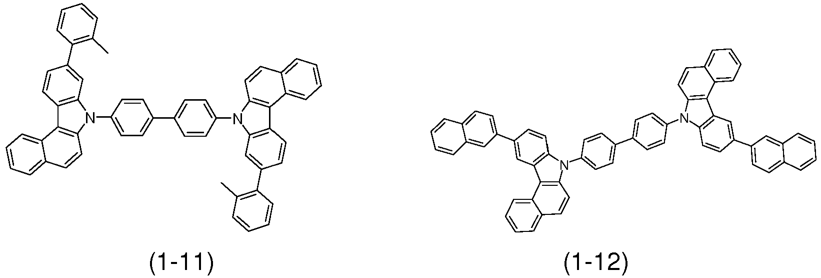

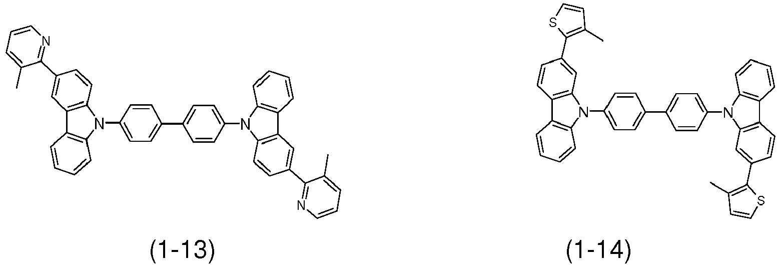

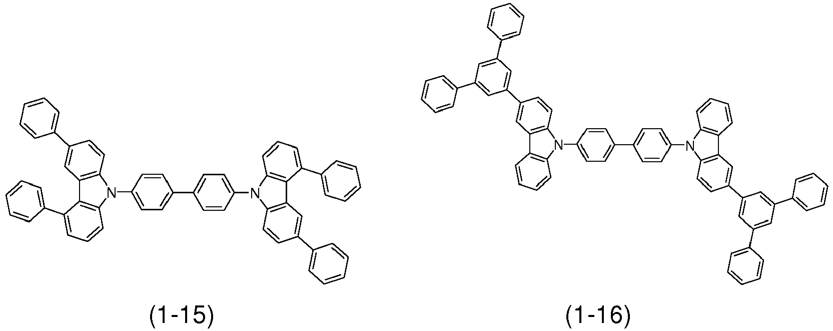

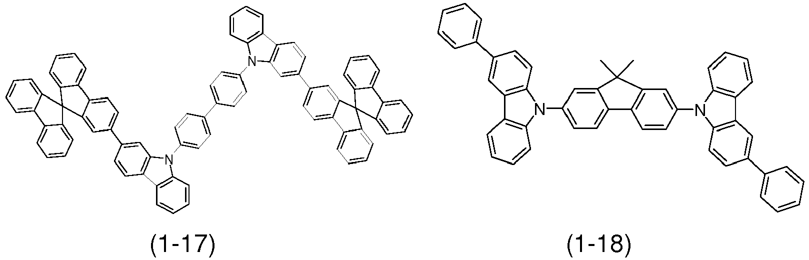

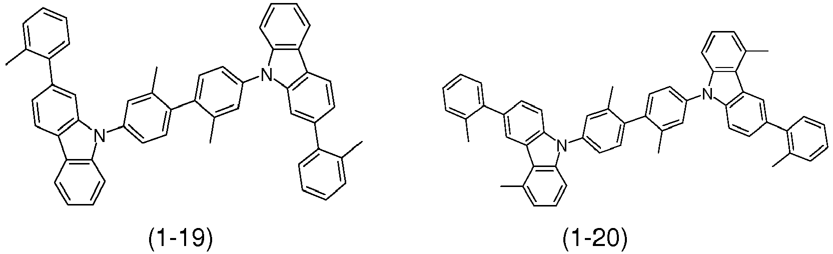

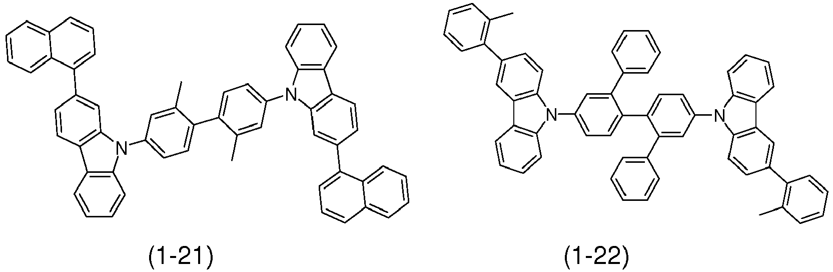

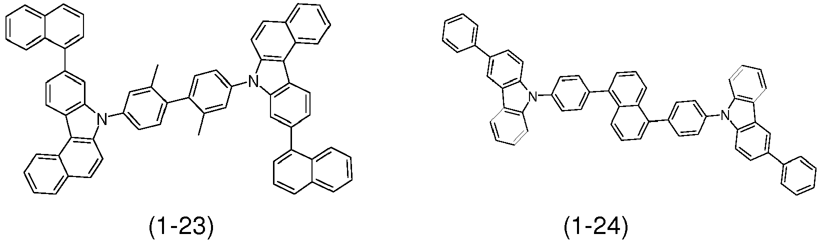

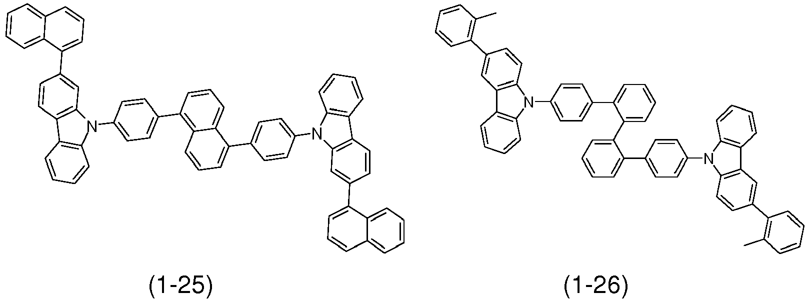

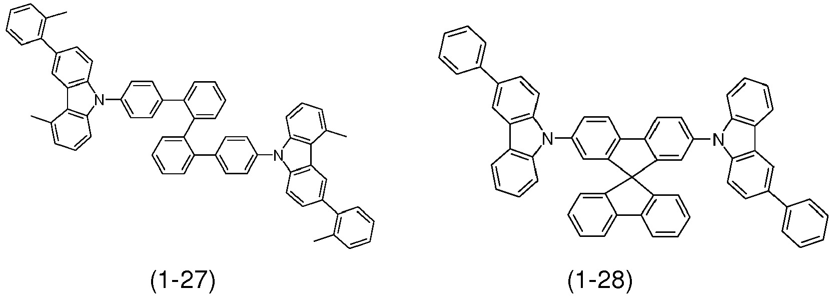

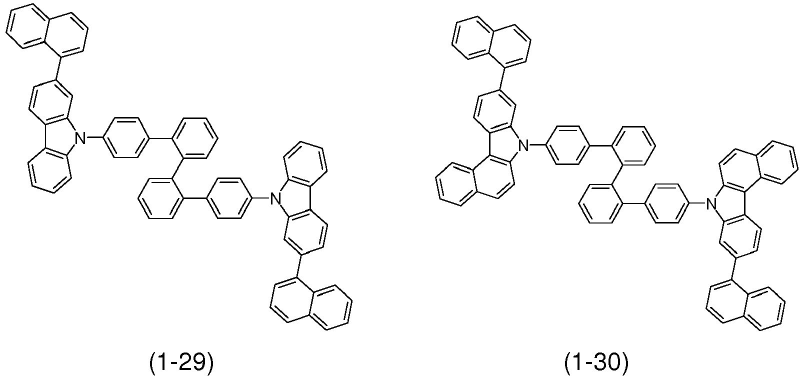

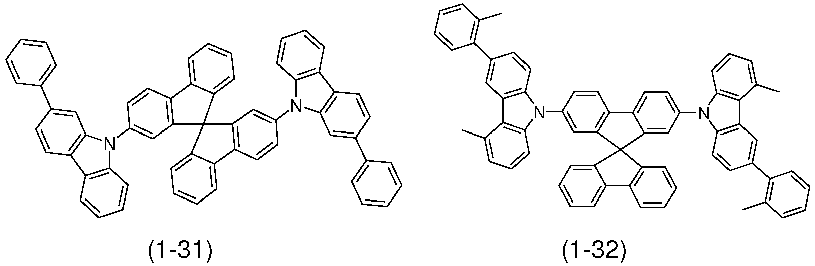

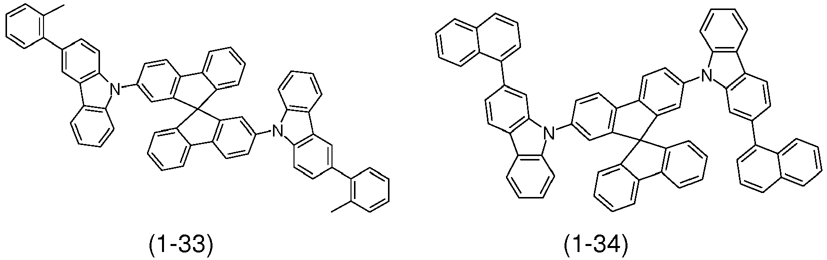

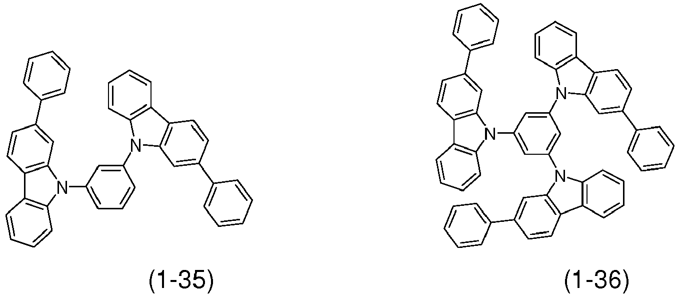

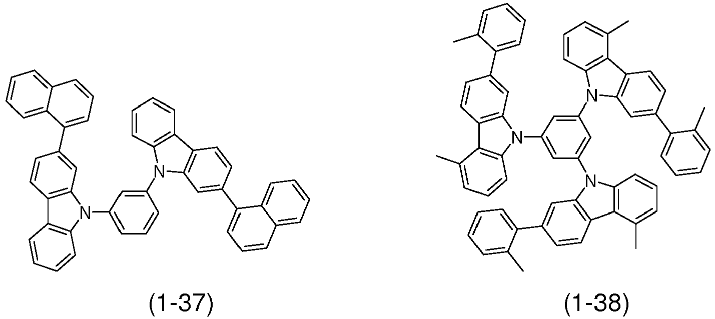

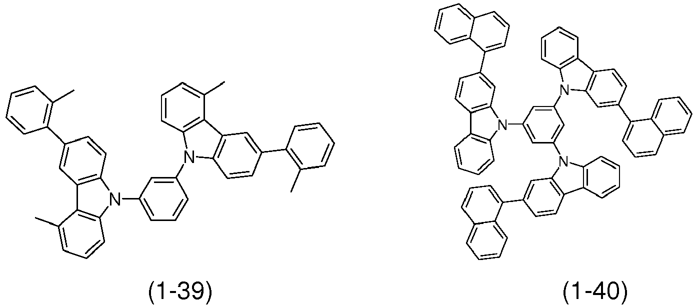

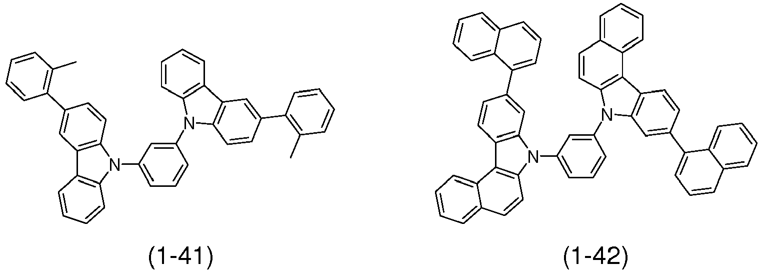

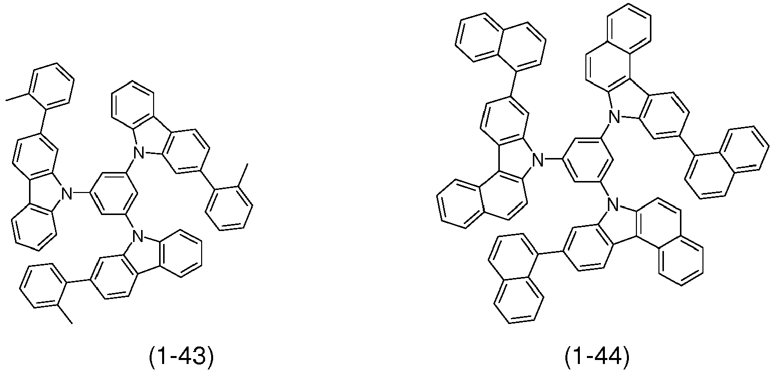

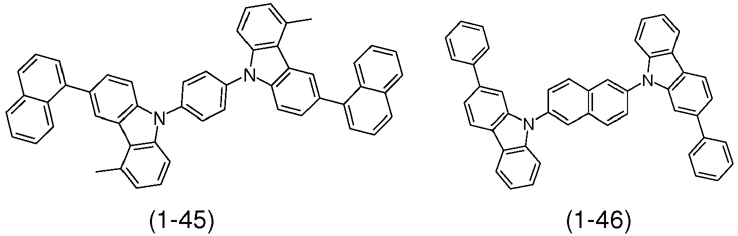

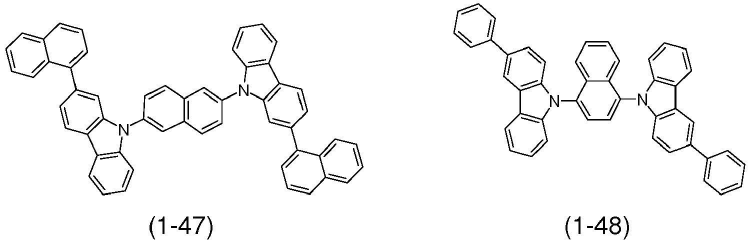

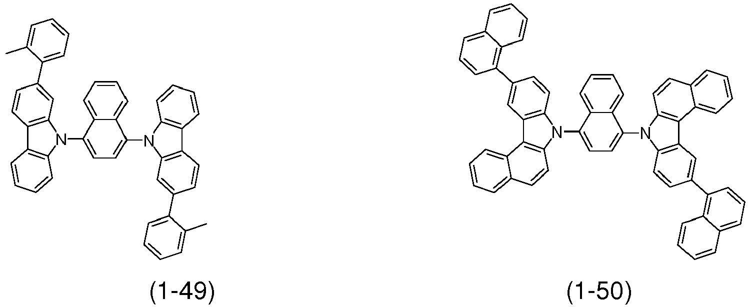

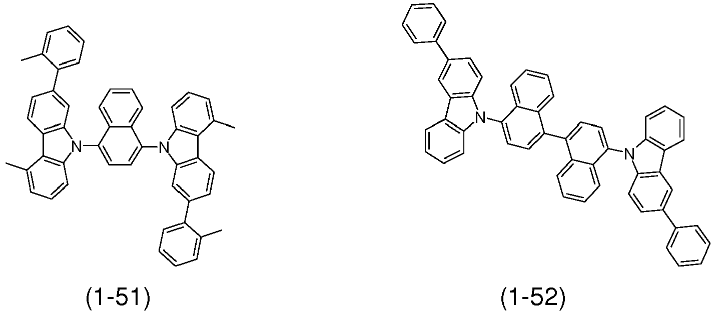

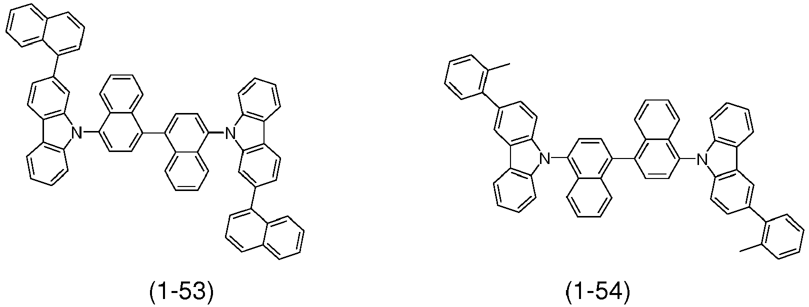

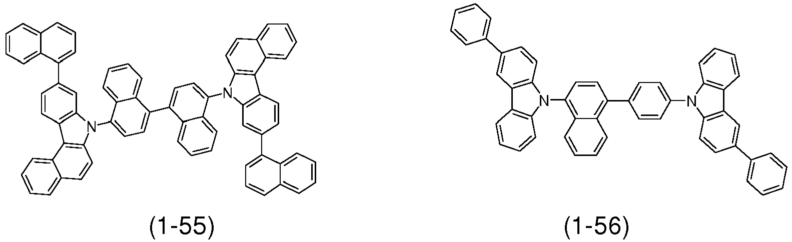

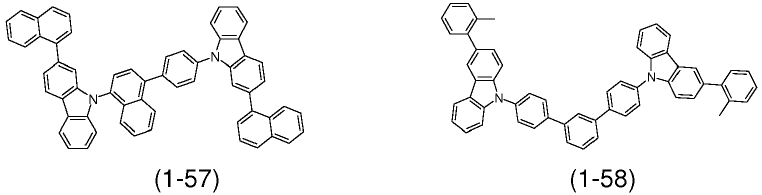

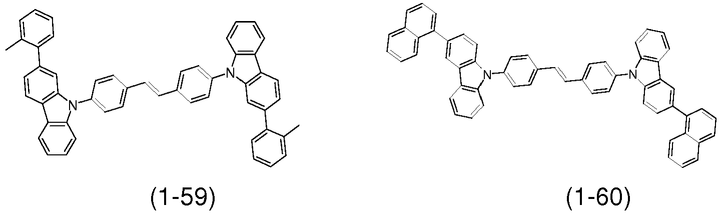

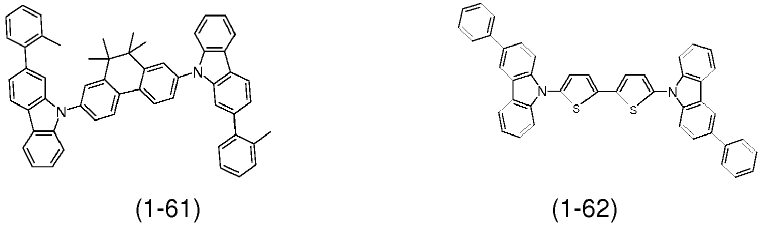

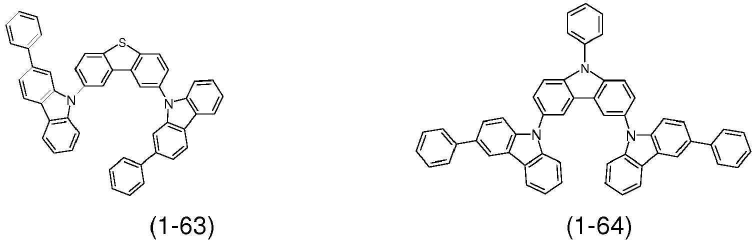

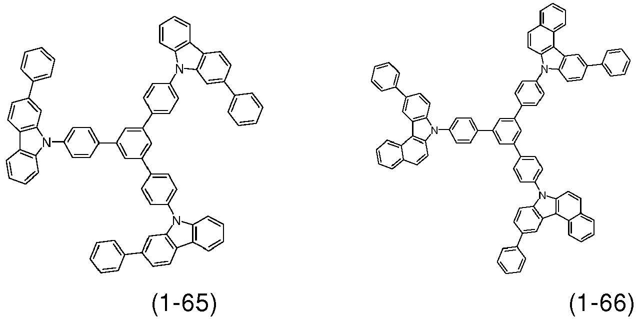

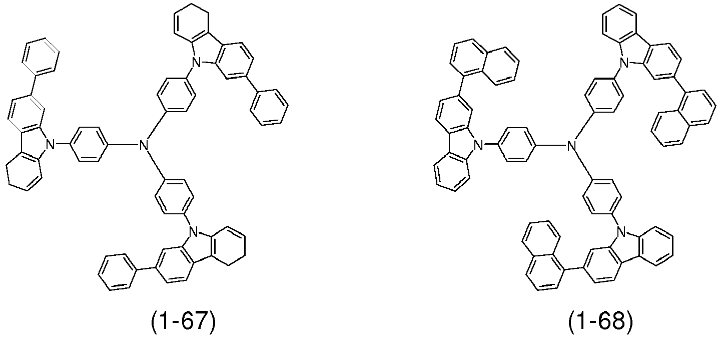

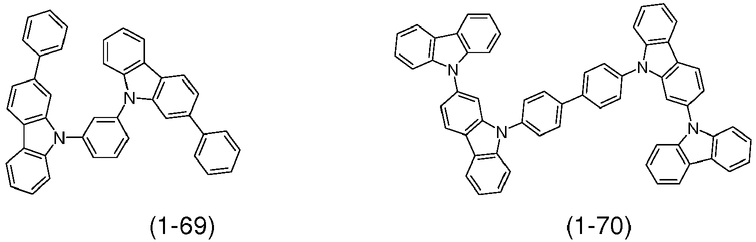

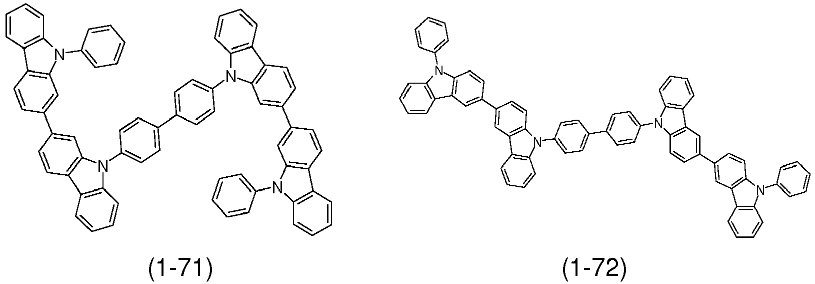

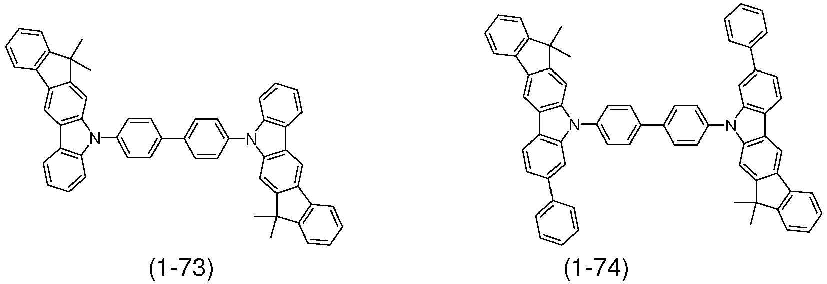

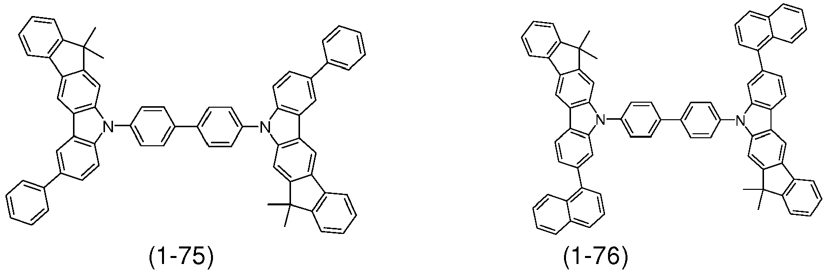

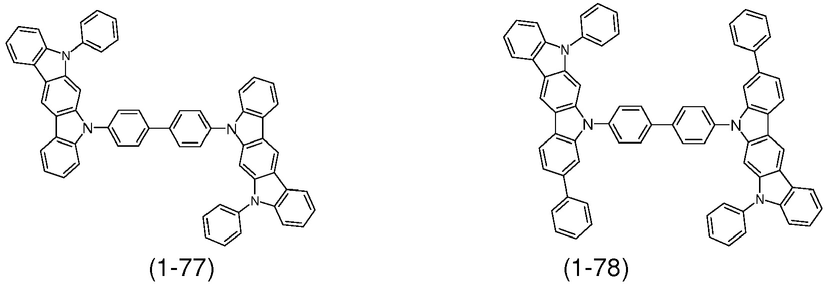

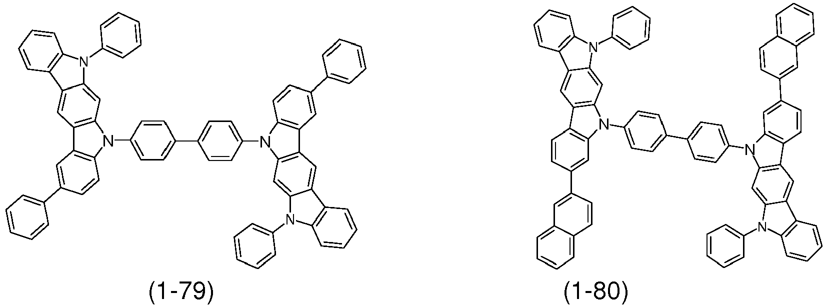

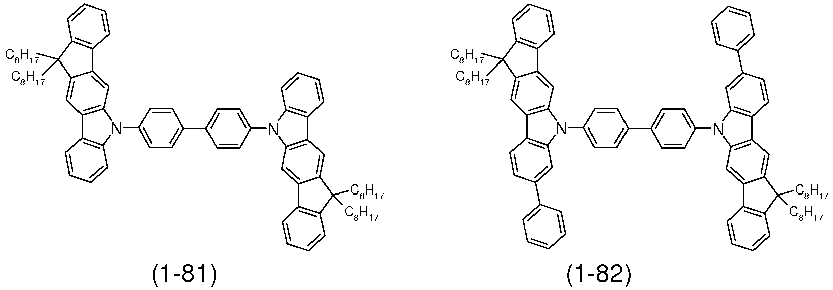

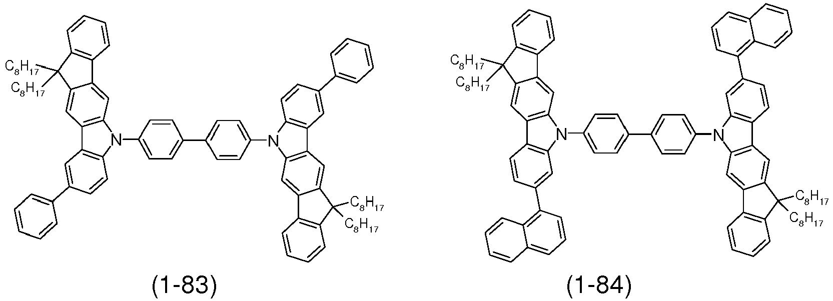

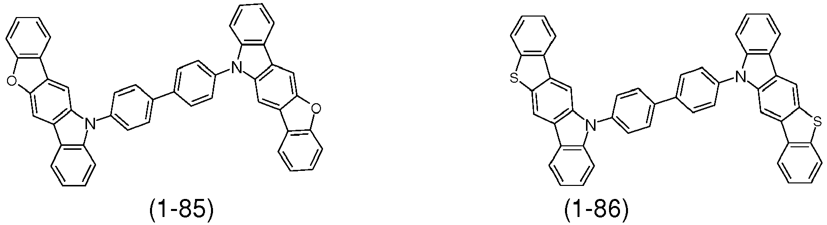

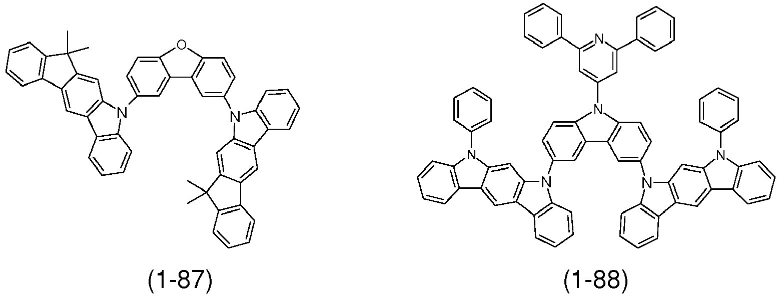

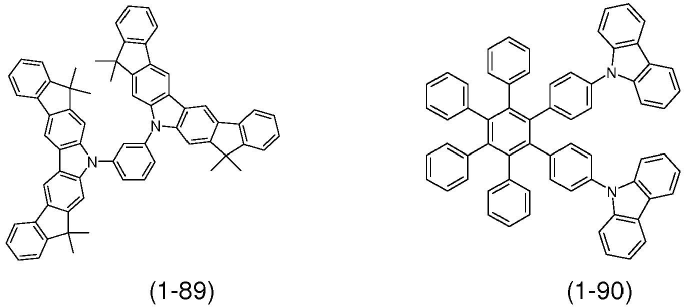

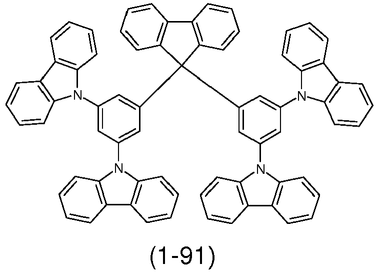

Die erfindungsgemäß eingesetzten Carbazolverbindungen können nach Standardmethoden der organischen Chemie synthetisiert werden, wie z.B. in der WO 2008/086851 ausführlich offenbart

So ist bekannt, dass 2-Nitrobiphenylderivate mit einem Trialkylphosphit zu den entsprechenden Carbazolderivaten umgesetzt werden können (M. Tavasli et al., Synthesis 2005, 1619-1624). Diese Reaktion lässt sich zum Aufbau von 2-Aryl-substituierten Carbazolderivaten verwenden, indem zunächst ein entsprechendes Aryl-substituiertes 2-Nitrobiphenylderivat aufgebaut wird, welches anschließend mit Trialkylphosphit umgesetzt wird. Das 2-Aryl-substituierte Carbazolderivat lässt sich mit einem Dibromaromaten in einer Hartwig-Buchwald-Kupplung unter Standardbedingungen zur Verbindung gemäß Formel (1) kuppeln. Die verschiedenen Ausführungsmethoden und Reaktionsbedingungen der Hartwig-Buchwald-Kupplung sind dem Fachmann der organischen Synthese bekannt. Statt eines Dibromaromaten lassen sich auch entsprechende Verbindungen mit anderen Abgangsgruppen, beispielsweise Chlor, lod, Triflat, Tosylat oder allgemein Sulfonaten, verwenden. Durch Verwendung von trisubstituierten Aromaten oder Verbindungen mit nochmals mehr Abgangsgruppen lassen sich entsprechend Verbindungen gemäß Formel (1) synthetisieren, in denen der Index q für 2 oder mehr steht.

Die Synthese von Verbindungen der Formel (1) ist im folgenden Schema 1 abgebildet, wobei der Übersichtlichkeit halber q = 1 gewählt wurde und keine Substituenten R oder R¹ abgebildet sind:

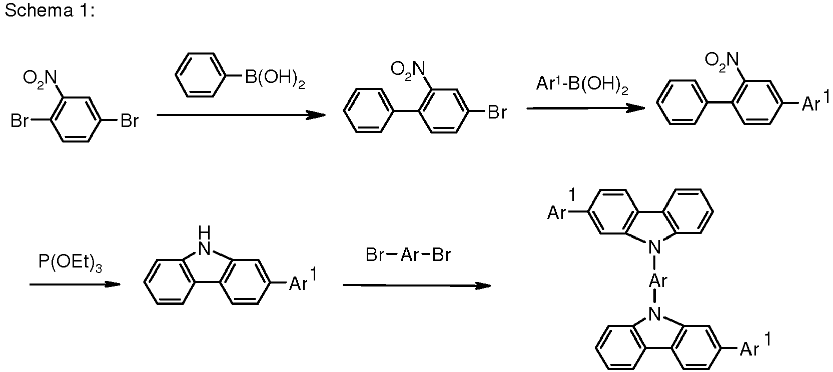

Die Referenz-Formulierung enthält gemäß einer Ausführungsform eine Ketonverbindung, bevorzugt eine aromatische Ketonverbindung. In einer Ausführungsform ist die Ketonverbindung vorzugsweise eine Verbindung der folgenden Formel (2), 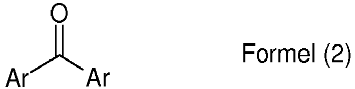 wobei für die verwendeten Symbole gilt:
- Ar: ist bei jedem Auftreten gleich oder verschieden ein aromatisches oder heteroaromatisches Ringsystem mit 5 bis 60 aromatischen Ringatomen, welches jeweils mit einer oder mehreren Gruppen R¹ substituiert sein kann;
- R¹: ist bei jedem Auftreten gleich oder verschieden H, D, F, Cl, Br, I, CHO, C(=O)Ar¹, P(=O)(Ar¹)₂, S(=O)Ar¹, S(=O)₂Ar¹, CR²=CR²Ar¹, CN, NO₂, Si(R²)₃, B(OR²)₂, B(R²)₂, B(N(R²)₂)₂, OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches, aromatisches oder heteroaromatisches Ringsystem bilden;
- Ar¹: ist bei jedem Auftreten gleich oder verschieden ein aromatisches oder heteroaromatisches Ringsystem mit 5 bis 40 aromatischen Ringatomen, das mit einem oder mehreren Resten R² substituiert sein kann;
- R²: ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches, aromatisches oder heteroaromatisches Ringsystem bilden.

Geeignete Verbindungen gemäß Formel (2) sind insbesondere die in der WO 04/093207 und in der DE 102008033943 offenbarten Ketone Aus der Definition der Verbindung der Formel (2) geht hervor, dass diese nicht nur eine Carbonylgruppe enthalten muss, sondern auch mehrere dieser Gruppen enthalten kann.

Bevorzugt ist die Gruppe Ar in den Ketonverbindungen der Formel (2) ein aromatisches Ringsystem mit 6 bis 40 aromatischen Ringatomen, d.h. sie enthält keine Heteroarylgruppen. Wie oben definiert, muss das aromatische Ringsystem nicht notwendigerweise nur aromatische Gruppen aufweisen, sondern es können auch zwei Arylgruppen durch eine nichtaromatische Gruppe, beispielsweise durch eine weitere Carbonylgruppe unterbrochen sein.

In einer weiteren bevorzugten Ausführungsform weist die Gruppe Ar der Ketonverbindung nicht mehr als zwei kondensierte Ringe auf. Sie ist also vorzugsweise nur aus Phenyl- und/oder Naphthylgruppen, besonders bevorzugt nur aus Phenylgruppen, aufgebaut, enthält aber keine größeren kondensierten Aromaten, wie beispielsweise Anthracen.

Bevorzugte Gruppen Ar, die an die Carbonylgruppe der Ketonverbindung gebunden sind, sind Phenyl, 2-, 3- oder 4-Tolyl, 3- oder 4-o-Xylyl, 2- oder 4-m-Xylyl, 2-p-Xylyl, o-, m- oder p-tert-Butylphenyl, o-, m- oder p-Fluorphenyl, Benzophenon, 1-, 2- oder 3-Phenylmethanon, 2-, 3- oder 4-Biphenyl, 2-, 3- oder 4-o-Terphenyl, 2-, 3- oder 4-m-Terphenyl, 2-, 3- oder 4-p-Terphenyl, 2'-p-Terphenyl, 2'-, 4'- oder 5'-m-Terphenyl, 3'- oder 4'-o-Terphenyl, p-, m,p-, o,p-, m,m-, o,m- oder o,o-Quaterphenyl, Quinquephenyl, Sexiphenyl, 1-, 2-, 3- oder 4-Fluorenyl, 2-, 3- oder 4-Spiro-9,9'-bifluorenyl, 1-, 2-, 3- oder 4-(9,10-Dihydro)phenanthrenyl, 1- oder 2-Naphthyl, 2-, 3-, 4-, 5-, 6-, 7- oder 8-Chinolinyl, 1-, 3-, 4-, 5-, 6-, 7- oder 8-iso-Chinolinyl, 1- oder 2-(4-Methylnaphthyl), 1- oder 2-(4-Phenylnaphthyl), 1- oder 2-(4-naphthyl-naphthyl), 1-, 2- oder 3-(4-naphthyl-phenyl), 2-, 3- oder 4-Pyridyl, 2-, 4- oder 5-Pyrimidinyl, 2- oder 3-Pyrazinyl, 3- oder 4-Pyridanzinyl, 2-(1,3,5-Triazin)yl-, 2-, 3- oder 4-(Phenylpyridyl), 3-, 4-, 5- oder 6-(2,2'-Bipyridyl), 2-, 4-, 5- oder 6-(3,3'-Bipyridyl), 2- oder 3-(4,4'-Bipyridyl) und Kombinationen eines oder mehrerer dieser Reste.

Die Gruppen Ar können, wie oben beschrieben, durch einen oder mehrere Reste R¹ substituiert sein. Diese Reste R¹ der Ketonverbindung sind vorzugsweise gleich oder verschieden bei jedem Auftreten ausgewählt aus der Gruppe bestehend aus H, F, C(=O)Ar¹, P(=O)(Ar¹)₂, S(=O)Ar¹, S(=O)₂Ar¹, einer geradkettigen Alkylgruppe mit 1 bis 4 C-Atomen oder einer verzweigten oder cyclischen Alkylgruppe mit 3 bis 5 C-Atomen, die jeweils mit einem oder mehreren Resten R² substituiert sein kann, wobei ein oder mehrere H-Atome durch F ersetzt sein können, oder einem aromatischen Ringsystem mit 6 bis 24 aromatischen Ringatomen, das durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches, aromatisches oder heteroaromatisches Ringsystem bilden.

Da die Formulierung als Lösung aufgebracht wird, sind auch geradkettige, verzweigte oder cyclische Alkylgruppen mit bis zu 10 C-Atomen als Substituenten R¹ bevorzugt. Die Reste R¹ sind besonders bevorzugt gleich oder verschieden bei jedem Auftreten ausgewählt aus der Gruppe bestehend aus H, C(=O)Ar¹ oder einem aromatischen Ringsystem mit 6 bis 24 aromatischen Ringatomen, das durch einen oder mehrere Reste R² substituiert sein kann, vorzugsweise aber unsubstituiert ist.

In einer weiteren bevorzugten Ausführungsform ist die Gruppe Ar¹ der Ketonverbindung gleich oder verschieden bei jedem Auftreten ein aromatisches Ringsystem mit 6 bis 24 aromatischen Ringatomen, das mit einem oder mehreren Resten R² substituiert sein kann. Besonders bevorzugt ist Ar¹ gleich oder verschieden bei jedem Auftreten ein aromatisches Ringsystem mit 6 bis 12 aromatischen Ringatomen.

Besonders bevorzugt sind Benzophenon-Derivate, die jeweils an den 3,5,3',5'-Positionen durch ein aromatisches oder heteroaromatisches Ringsystem mit 5 bis 30 aromatischen Ringatomen substituiert sind, welche wiederum durch einen oder mehrere Reste R¹ gemäß der obigen Definition substituiert sein können. Weiterhin bevorzugt sind Ketone, welche mit mindestens einer Spirobifluorengruppe substituiert sind.

Bevorzugte aromatische Ketone sind daher die Verbindungen der folgenden Formel (3) bis (6), 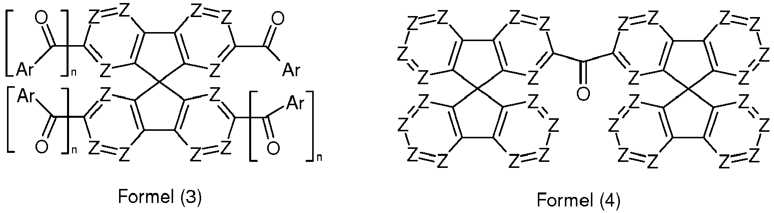 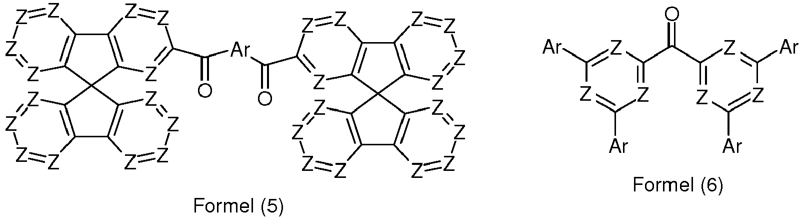 wobei Ar und R¹ dieselbe Bedeutung haben, wie oben bei der Ketonverbindung (Formel (2)) beschrieben, und weiterhin gilt:
- Z: ist gleich oder verschieden bei jedem Auftreten CR¹ oder N;
- n: ist gleich oder verschieden bei jedem Auftreten 0 oder 1.

Vorzugsweise steht Ar in den oben genannten Formeln (3), (5) und (6) für ein aromatisches oder heteroaromatisches Ringsystem mit 1 bis 30 aromatischen Ringatomen, welches mit einem oder mehreren Resten R¹ substituiert sein kann. Besonders bevorzugt sind die oben genannten Gruppen Ar.

Beispiele für bevorzugte Verbindungen gemäß Formel (2) sind die im Folgenden abgebildeten Verbindungen (2-1) bis (2-70).

| | |
|---|---|
| 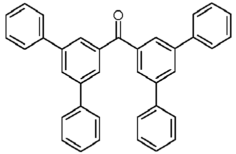 | 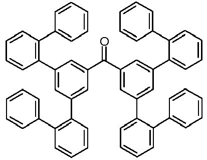 |
| (2-1) | (2-2) |
| 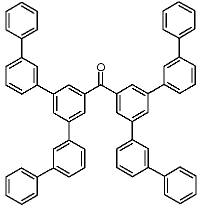 | 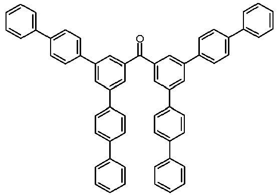 |
| (2-3) | (2-4) |
| 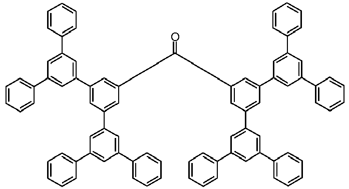 | 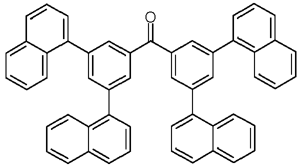 |
| (2-5) | (2-6) |
| 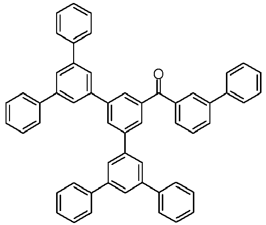 | 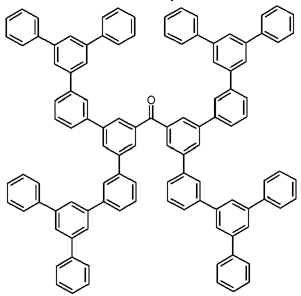 |
| (2-7) | (2-8) |
| 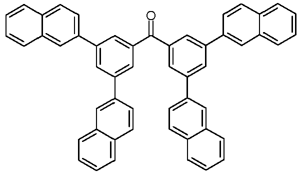 | 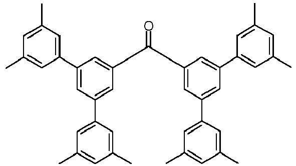 |
| (2-9) | (2-10) |
| 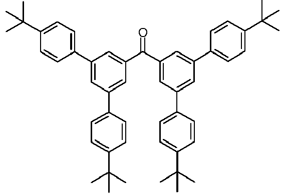 | 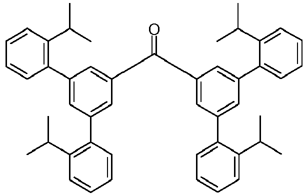 |
| (2-11) | (2-12) |
| 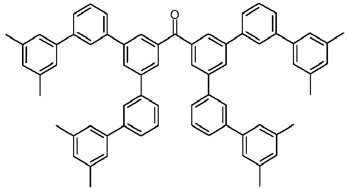 | 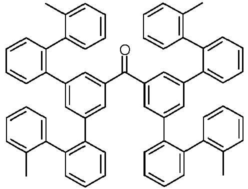 |
| (2-13) | (2-14) |
| 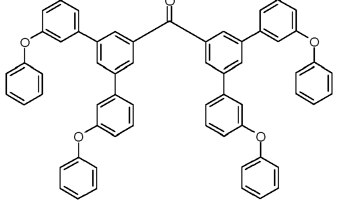 | 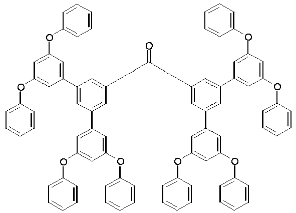 |
| (2-15) | (2-16) |
| 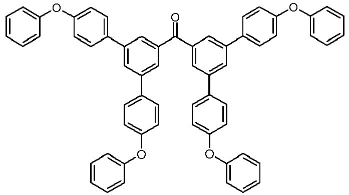 | 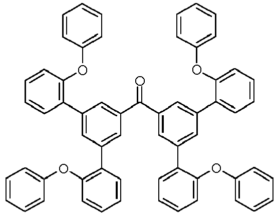 |
| (2-17) | (2-18) |
| 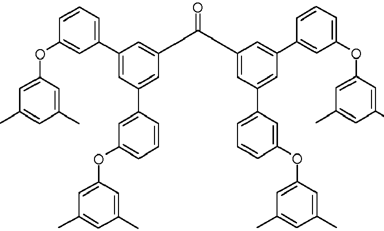 | 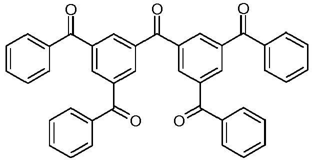 |
| (2-19) | (2-20) |
| 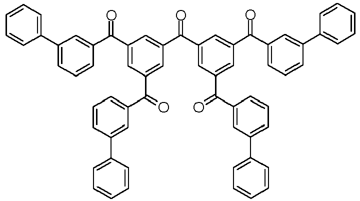 | 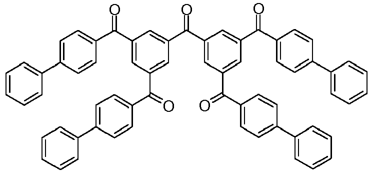 |
| (2-21) | (2-22) |
| 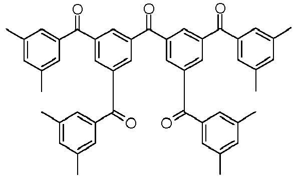 | 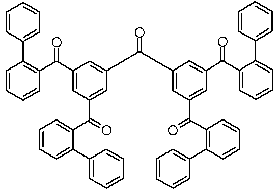 |
| (2-23) | (2-24) |
| 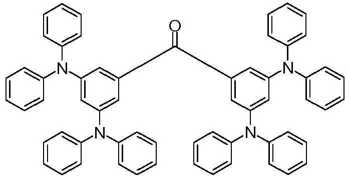 | 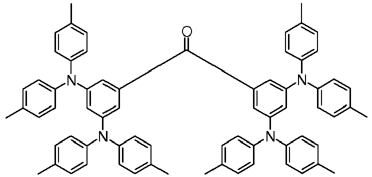 |
| (2-25) | (2-26) |
| 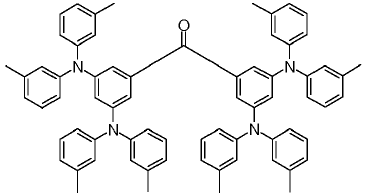 | 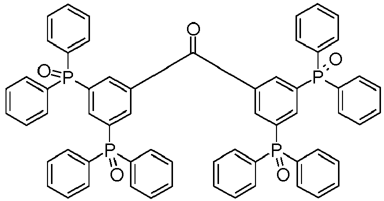 |
| (2-27) | (2-28) |
| 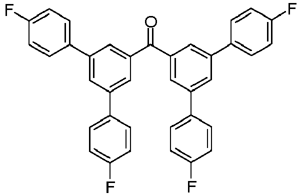 | 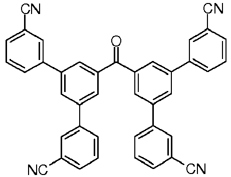 |
| (2-29) | (2-30) |
| 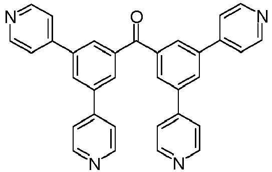 | 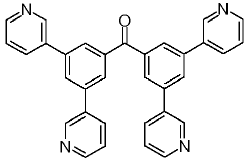 |
| (2-31) | (2-32) |
| 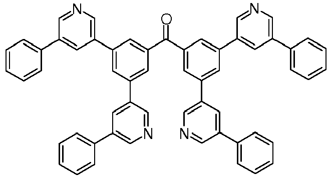 | 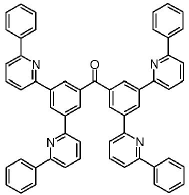 |
| (2-33) | (2-34) |
| 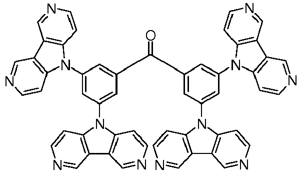 | 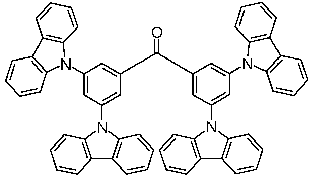 |
| (2-35) | (2-36) |
| 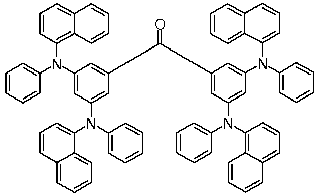 | 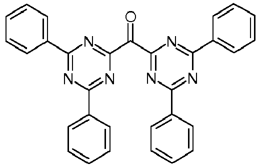 |
| (2-37) | (2-38) |
| 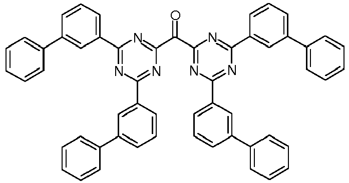 | 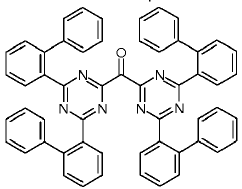 |
| (2-39) | (2-40) |
| 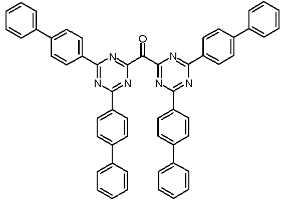 | 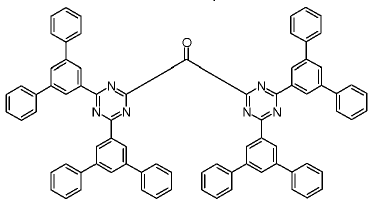 |
| (2-41) | (2-42) |
| 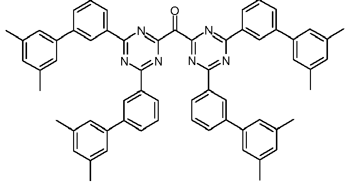 | 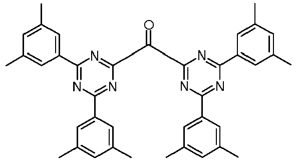 |
| (2-43) | (2-44) |
| 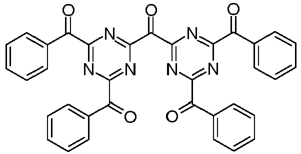 | 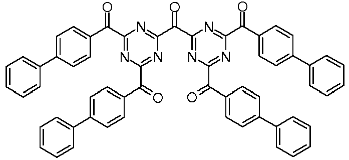 |
| (2-45) | (2-46) |
| 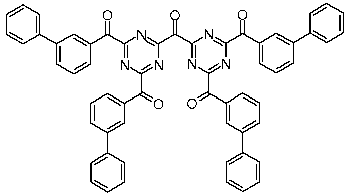 | 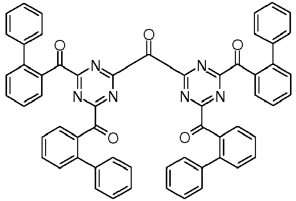 |
| (2-47) | (2-48) |
| 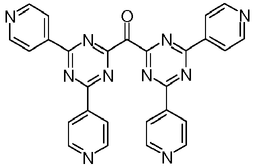 | 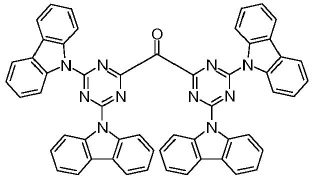 |
| (2-49) | (2-50) |
| 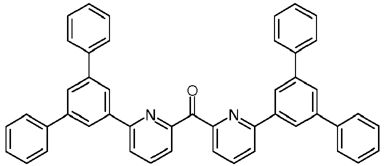 | 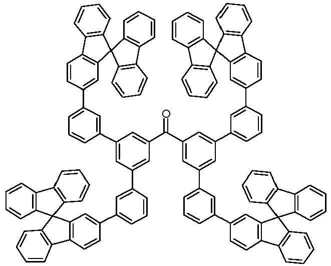 |
| (2-51) | (2-52) |
| 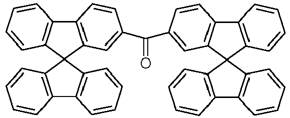 | 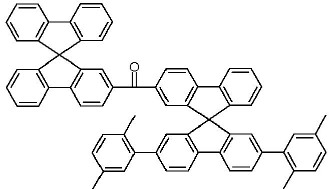 |
| (2-53) | (2-54) |
| 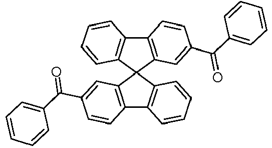 | 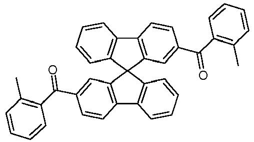 |
| (2-55) | (2-56) |
| 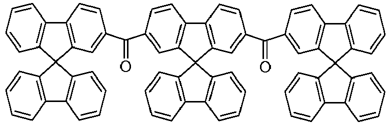 | 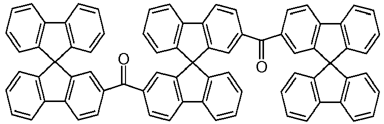 |
| (2-57) | (2-58) |
| 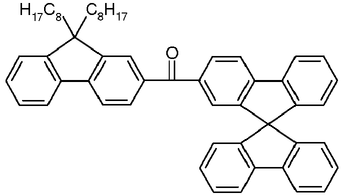 | 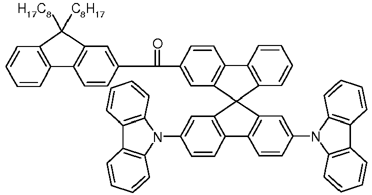 |
| (2-59) | (2-60) |
| 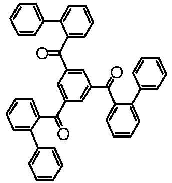 | 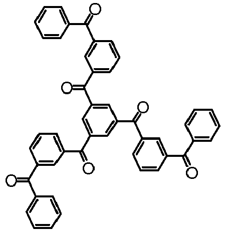 |
| (2-61) | (2-62) |
| 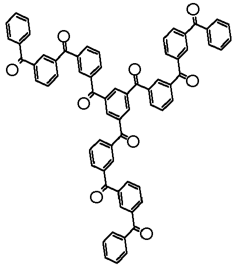 | 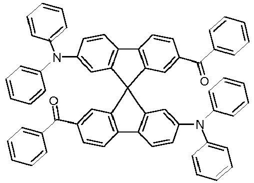 |
| (2-63) | (2-64) |
| 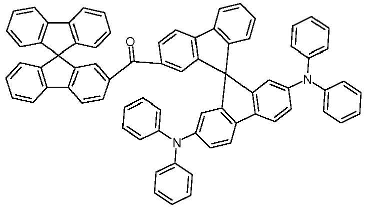 | 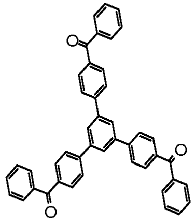 |
| (2-65) | (2-66) |
| 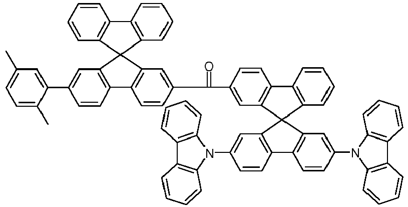 | 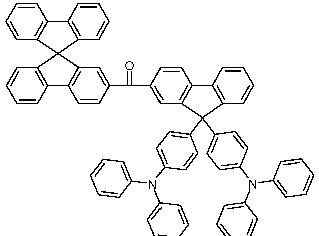 |
| (2-67) | (2-68) |
| 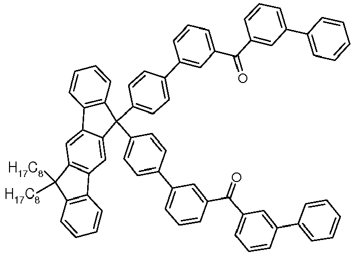 | 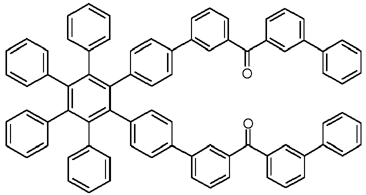 |
| (2-69) | (2-70) |

Geeignete Triazinderivate, die in der erfindungsgemäßen Formulierung verwendet werden können, sind insbesondere 1,3,5-Triazine, welche mit mindestens einer, vorzugsweise mindestens zwei, besonders bevorzugt mit drei aromatischen oder heteroaromatischen Ringsystemen substituiert sind. Besonders bevorzugt sind also Verbindungen der folgenden Formeln (7) und (8), 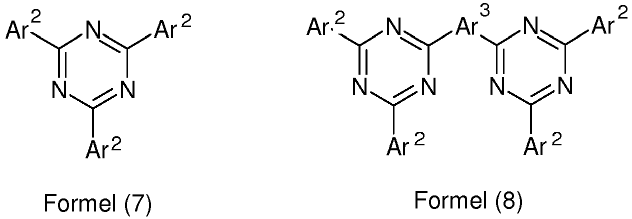 wobei
- Ar²: ist gleich oder verschieden bei jedem Auftreten ein monovalentes aromatisches oder heteroaromatisches Ringsystem mit 5 bis 60 aromatischen Ringatomen, welches jeweils mit einem oder mehreren Resten R¹ substituiert sein kann;
- Ar³: ist ein bivalentes aromatisches oder heteroaromatisches Ringsystem mit 5 bis 60 aromatischen Ringatomen, welches mit einem oder mehreren Resten R¹ substituiert sein kann;
und R¹ die oben in Bezug auf die Ketonverbindung der Formel (2) angegebene Bedeutung hat.

Vorzugsweise ist in den Verbindungen der Formeln (7) und (8) mindestens eine Gruppe Ar² ausgewählt aus den Gruppen der folgenden Formeln (9) bis (15), und die anderen Gruppen Ar² haben die für Formel (7) oder (8) angegebene Bedeutung, 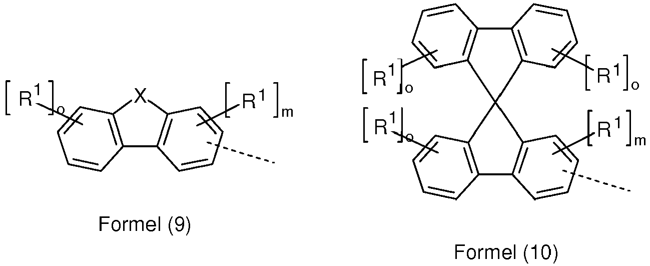 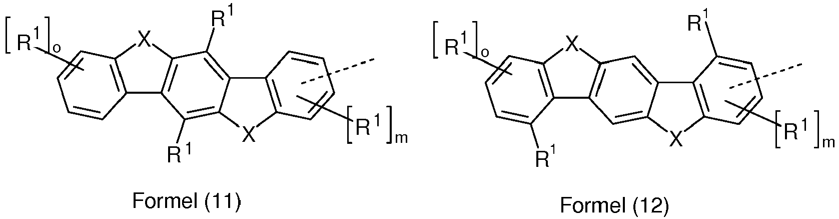 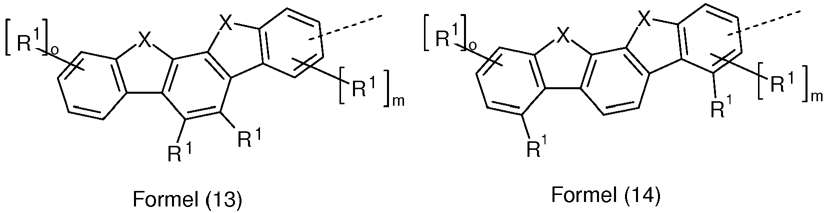 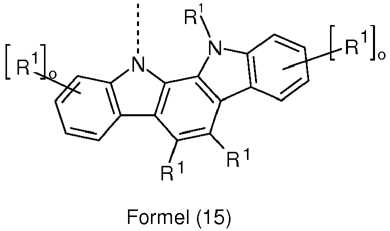 wobei R¹ dieselbe Bedeutung hat, wie oben beschrieben, die gestrichelte Bindung die Verknüpfung mit der Triazineinheit darstellt und weiterhin gilt:
- X: ist gleich oder verschieden bei jedem Auftreten eine bivalente Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹;
- m: ist bei jedem Auftreten gleich oder verschieden 0, 1, 2 oder 3;
- o: ist bei jedem Auftreten gleich oder verschieden 0, 1, 2, 3 oder 4.

Besonders bevorzugte Gruppen Ar² der Triazinverbindung sind ausgewählt aus den Gruppen der folgenden Formeln (9a) bis (15a), 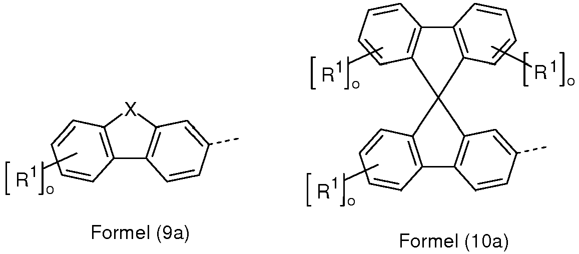 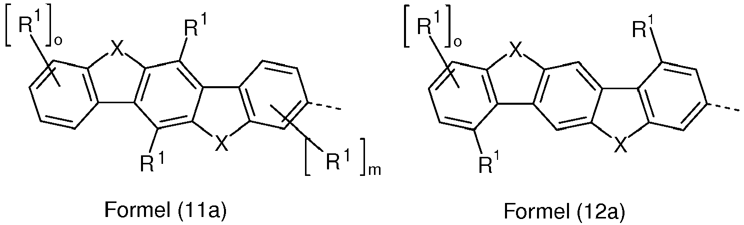 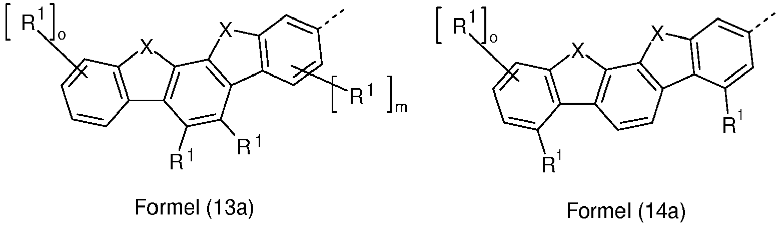 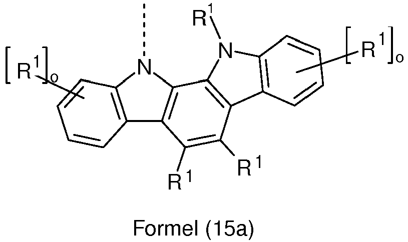 wobei die verwendeten Symbole und Indices dieselbe Bedeutung haben, wie oben beschrieben. Dabei ist X vorzugsweise gleich oder verschieden ausgewählt aus C(R¹)₂, N(R¹), O oder S, besonders bevorzugt C(R¹)₂.

Bevorzugte Gruppen Ar³ in den Verbindungen der Formel (8) sind ausgewählt aus den Gruppen der folgenden Formeln (16) bis (22), 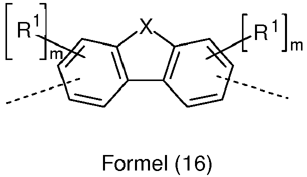 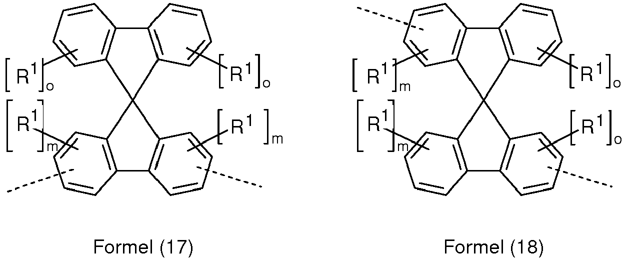 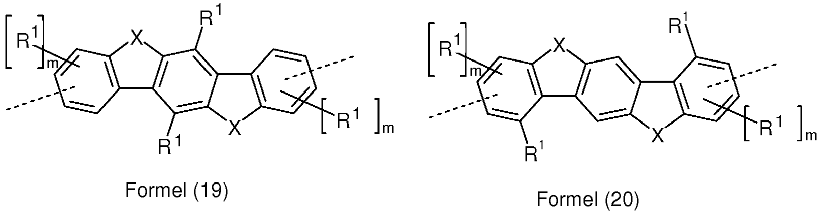 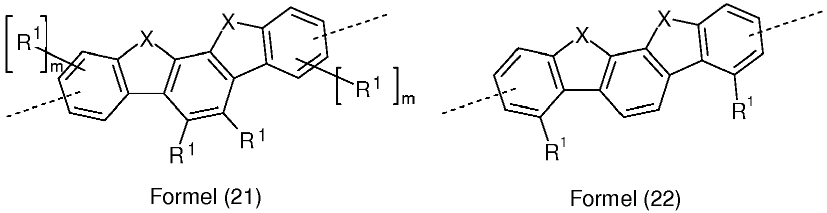 wobei die verwendeten Symbole und Indices dieselbe Bedeutung haben, wie oben in Bezug auf die Triazinverbindung beschrieben und die gestrichelte Bindungen die Verknüpfung mit den beiden Triazineinheiten darstellen.

Besonders bevorzugte Gruppen Ar³ sind ausgewählt aus den folgenden Formeln (16a) bis (22a), 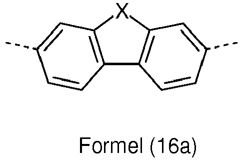 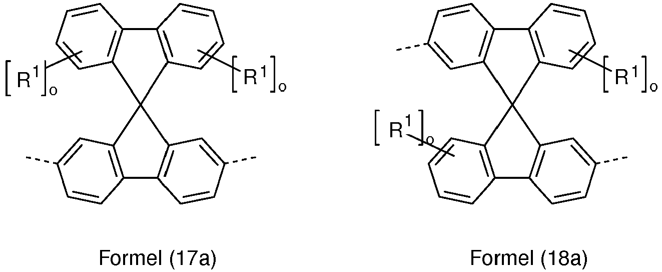 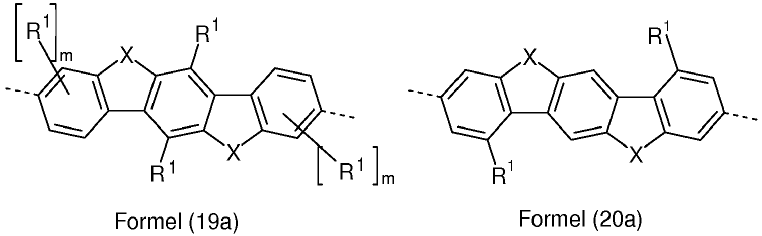 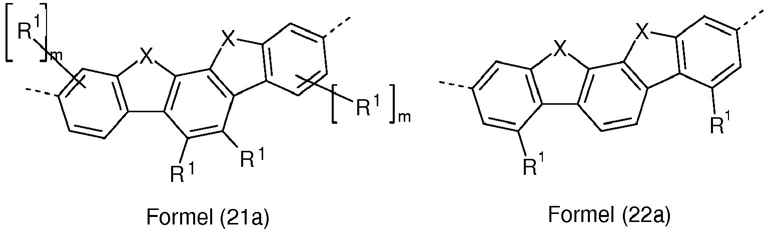 wobei die verwendeten Symbole und Indices dieselbe Bedeutung haben, wie oben in Bezug auf die Triazinverbindung beschrieben. Dabei ist X vorzugsweise gleich oder verschieden ausgewählt aus C(R¹)₂, N(R¹), O oder S, besonders bevorzugt C(R¹)₂.

Bevorzugt sind weiterhin Verbindungen der oben aufgeführten Formel (8), in der die Gruppe Ar³ aus den oben aufgeführten Formeln (16) bis (22) ausgewählt ist und Ar² gleich oder verschieden bei jedem Auftreten ausgewählt ist aus den oben aufgeführten Formeln (9) bis (15) oder Phenyl, 1- oder 2-Naphthyl, ortho-, meta- oder para-Biphenyl, welche durch einen oder mehrere Reste R¹ substituiert sein können, jedoch vorzugsweise unsubstituiert sind.

Beispiele für weitere bevorzugte Verbindungen gemäß der Formel (7) sind die im Folgenden abgebildeten Verbindungen (7-1) bis (7-65).

| | | |
|---|---|---|
| 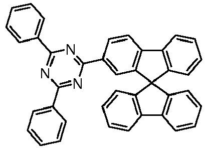 | 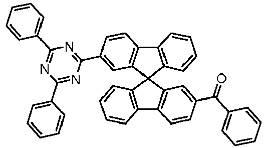 | 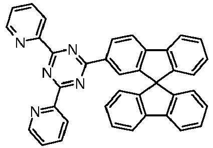 |
| (7-1) | (7-2) | (7-3) |
| 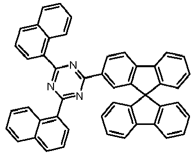 | 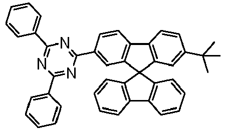 | 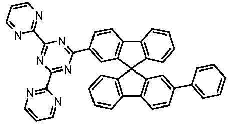 |
| (7-4) | (7-5) | (7-6) |
| 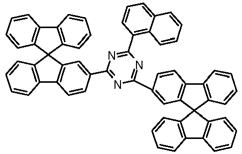 | 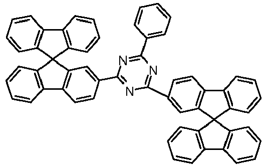 | 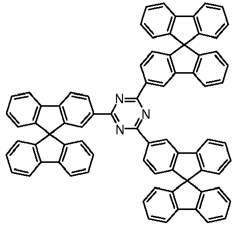 |
| (7-7) | (7-8) | (7-9) |
| 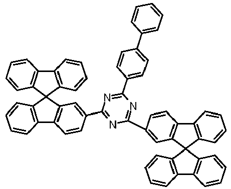 | 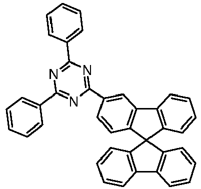 | 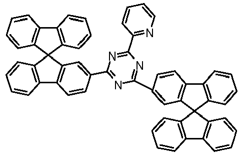 |
| (7-10) | (7-11) | (7-12) |
| 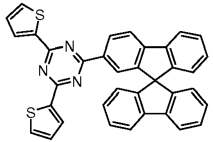 | 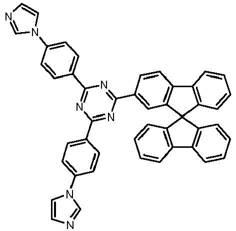 | 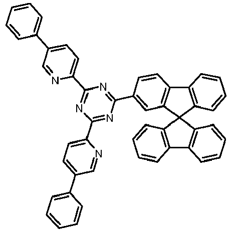 |
| (7-13) | (7-14) | (7-15) |
| 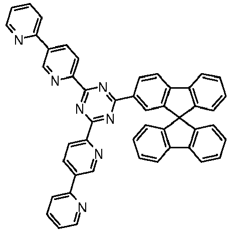 | 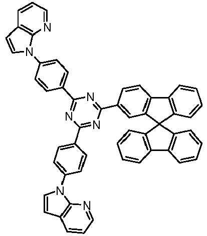 | 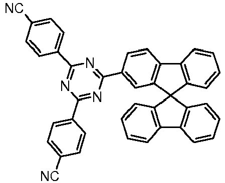 |
| (7-16) | (7-17) | (7-18) |
| 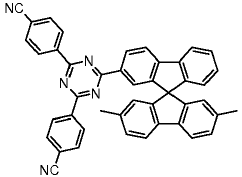 | 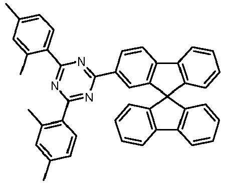 | 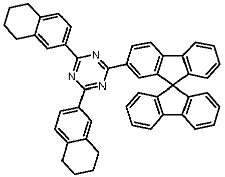 |
| (7-19) | (7-20) | (7-21) |
| 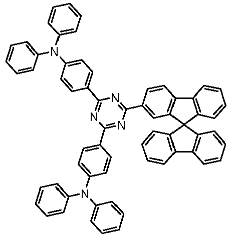 | 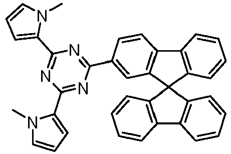 | 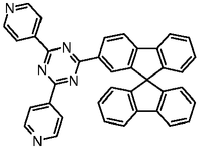 |
| (7-22) | (7-23) | (7-24) |
| 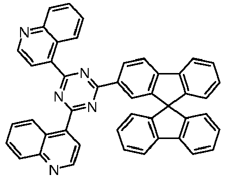 | 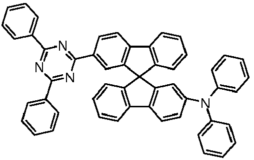 | 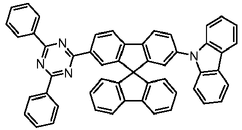 |
| (7-25) | (7-26) | (7-27) |
| 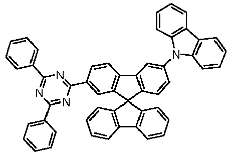 | 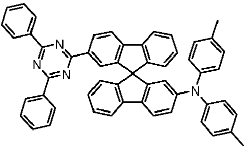 | 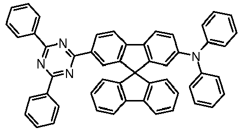 |
| (7-28) | (7-29) | (7-30) |
| 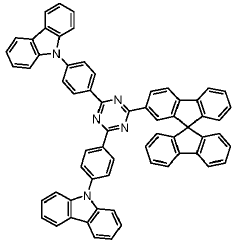 | 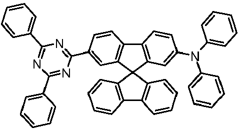 | 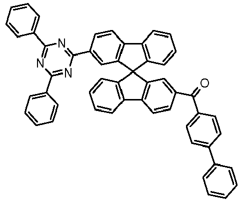 |
| (7-31) | (7-32) | (7-33) |
| 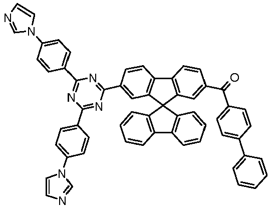 | 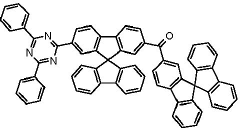 | 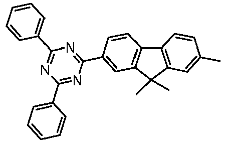 |
| (7-34) | (7-35) | (7-36) |
| 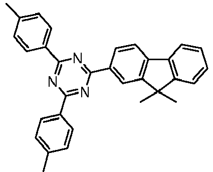 | 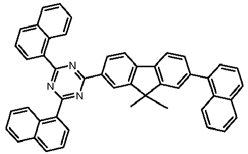 | 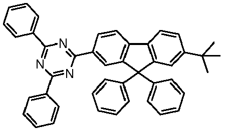 |
| (7-37) | (7-38) | (7-39) |
| 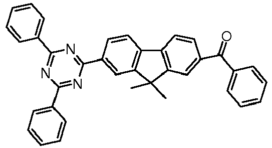 | 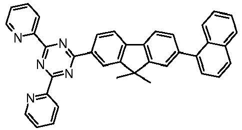 | 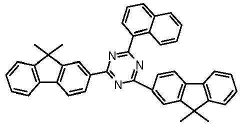 |
| (7-40) | (7-41) | (7-42) |
| 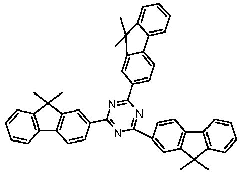 | 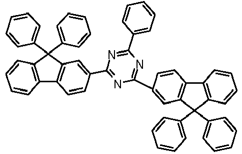 | 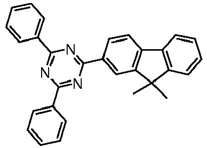 |
| (7-43) | (7-44) | (7-45) |
| 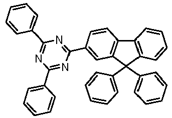 | 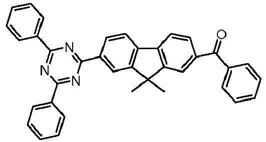 | 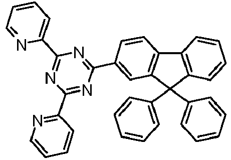 |
| (7-46) | (7-47) | (7-48) |
| 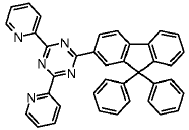 | 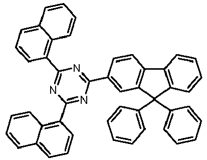 | 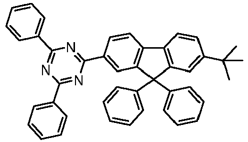 |
| (7-49) | (7-50) | (7-51) |
| 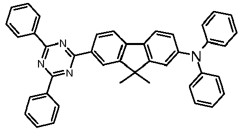 | 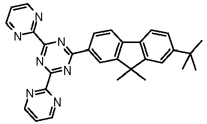 | 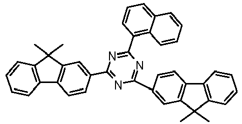 |
| (7-52) | (7-53) | (7-54) |
| 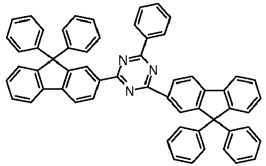 | 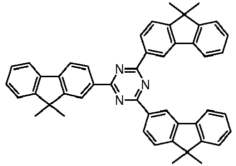 | 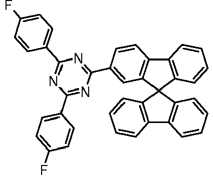 |
| (7-55) | (7-56) | (7-57) |
| 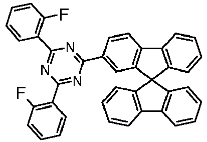 | 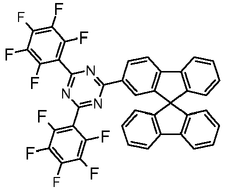 | 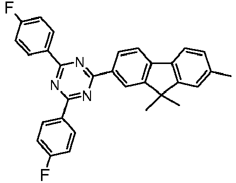 |
| (7-58) | (7-59) | (7-60) |
| 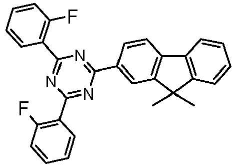 | 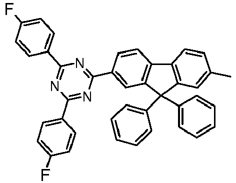 | 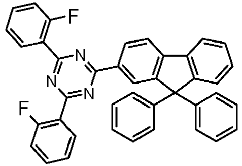 |
| (7-61) | (7-62) | (7-63) |
| 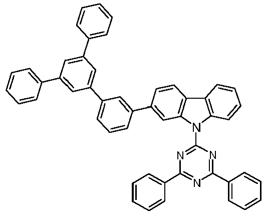 | 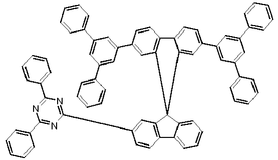 | |
| (7-64) | (7-65) | |

Beispiele für weitere bevorzugte Verbindungen gemäß der Formel (8) sind die im Folgenden abgebildeten Verbindungen (8-1) bis (8-16).

| | | |
|---|---|---|
| 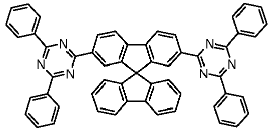 | 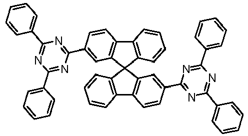 | 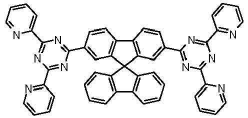 |
| (8-1) | (8-2) | (8-3) |
| 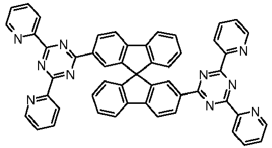 | 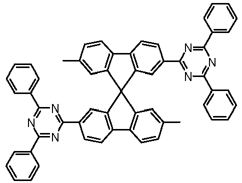 | 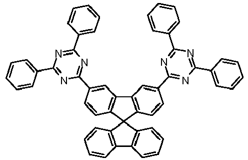 |
| (8-4) | (8-5) | (8-6) |
| 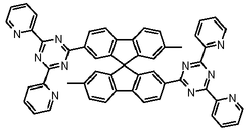 | 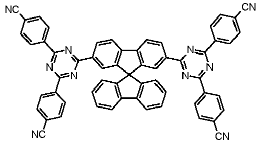 | 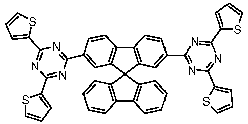 |
| (8-7) | (8-8) | (8-9) |
| 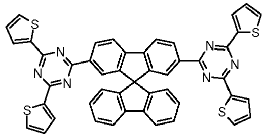 | 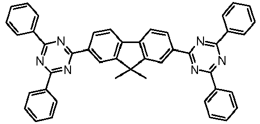 | 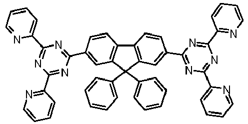 |
| (8-10) | (8-11) | (8-12) |
| 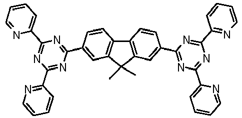 | 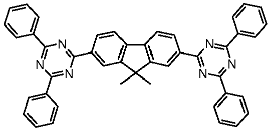 | 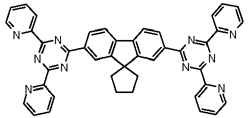 |
| (8-13) | (8-14) | (8-15) |
| 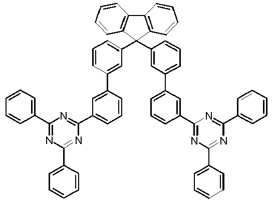 | | |
| (8-16) | | |

Wie oben bereits ausgeführt enthält die erfindungsgemäße Formulierung auch eine Triplett-Emitter-Verbindung. Eine Triplett-Emitter-Verbindung (phosphoreszierende Verbindung) gemäß der vorliegenden Erfindung ist eine Verbindung, welche bei Raumtemperatur Lumineszenz aus einem angeregten Zustand mit höherer Spinmultiplizität zeigt, also einem Spinzustand > 1, insbesondere aus einem angeregten Triplettzustand. Gemäß der vorliegenden Erfindung sollen alle lumineszierenden Übergangsmetallkomplexe mit Übergangsmetallen der zweiten und dritten Übergangsmetallreihe, insbesondere alle lumineszierenden Iridium- und Platinverbindungen als phosphoreszierende Verbindungen angesehen werden.

In einer bevorzugten Ausführungsform der vorliegenden Erfindung handelt es sich bei der Triplett-Emitter-Verbindung um eine rot phosphoreszierende Verbindung oder um eine grün phosphoreszierende Verbindung.

Als Triplett-Emitter-Verbindung (phosphoreszierende Verbindung) eignen sich insbesondere Verbindungen, die bei geeigneter Anregung Licht, vorzugsweise im sichtbaren Bereich, emittieren und außerdem mindestens ein Atom der Ordnungszahl größer 20, vorzugsweise größer 38 und kleiner 84, besonders bevorzugt größer 56 und kleiner 80 enthalten. Vorzugsweise werden als Triplett-Emitter-Verbindungen Verbindungen verwendet, die Kupfer, Molybdän, Wolfram, Rhenium, Ruthenium, Osmium, Rhodium, Iridium, Palladium, Platin, Silber, Gold oder Europium enthalten, insbesondere Verbindungen, die Iridium oder Platin enthalten.

Besonders bevorzugte erfindungsgemäße Formulierungen enthalten als Triplett-Emitter-Verbindung mindestens eine Verbindung der Formeln (23) bis (26), 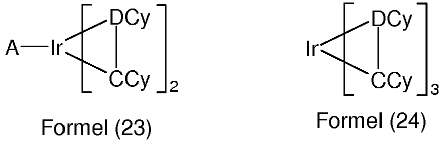 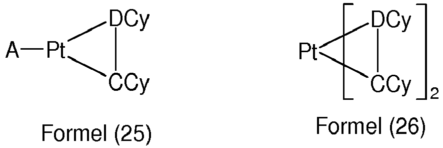 wobei R¹ dieselbe Bedeutung hat, wie oben in Bezug auf Formel (2) beschrieben, und für die weiteren verwendeten Symbole gilt:
- DCy: ist gleich oder verschieden bei jedem Auftreten eine cyclische Gruppe, die mindestens ein Donoratom, vorzugsweise Stickstoff, Kohlenstoff in Form eines Carbens oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
- CCy: ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann;
- A: ist gleich oder verschieden bei jedem Auftreten ein monoanionischer, zweizähnig chelatisierender Ligand, vorzugsweise ein Diketonatligand.

Dabei kann durch Bildung von Ringsystemen zwischen mehreren Resten R¹ auch eine Brücke zwischen den Gruppen DCy und CCy vorliegen. Weiterhin kann durch Bildung von Ringsystemen zwischen mehreren Resten R¹ auch eine Brücke zwischen zwei oder drei Liganden CCy-DCy bzw. zwischen ein oder zwei Liganden CCy-DCy und dem Liganden A vorliegen, so dass es sich um ein polydentates bzw. polypodales Ligandensystem handelt.

Beispiele der oben beschriebenen Emitter können der WO 00/70655, der WO 01/41512, der WO 02/02714, der WO 02/15645, der EP 1191613, der EP 1191612, der EP 1191614, der WO 04/081017, der WO 05/033244, der WO 05/042550, der WO 05/113563, der WO 06/008069, der WO 06/061182, der WO 06/081973, der DE 102008015526, der DE 102008027005 und der DE 102009007038 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Verbindungen verwenden.

Insbesondere ist dem Fachmann bekannt, welche phosphoreszierenden Komplexe mit welcher Emissionsfarbe emittieren.

Bevorzugte phosphoreszierende Verbindungen sind die in der folgenden Tabelle aufgeführten Strukturen (T-1) bis (T-140).

| | | |
|---|---|---|
| 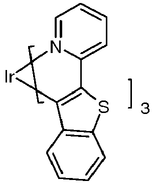 | 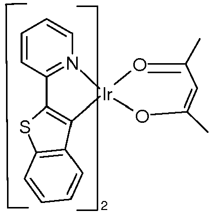 | 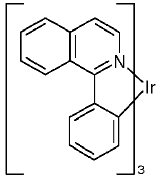 |
| (T-1) | (T-2) | (T-3) |
| 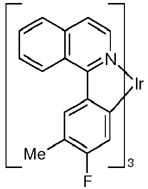 | 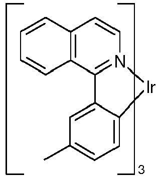 | 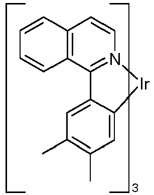 |
| (T-4) | (T-5) | (T-6) |
| 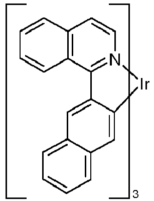 | 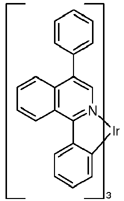 | 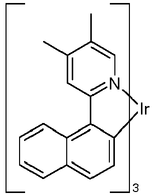 |
| (T-7) | (T-8) | (T-9) |
| 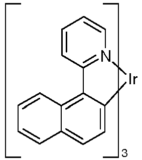 | 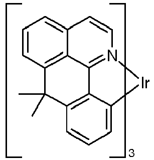 | 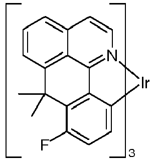 |
| (T-10) | (T-11) | (T-12) |
| 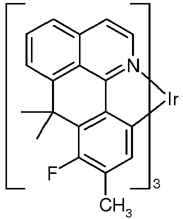 | 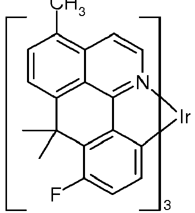 | 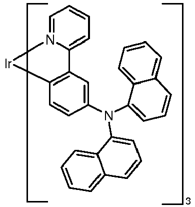 |
| (T-13) | (T-14) | (T-15) |
| 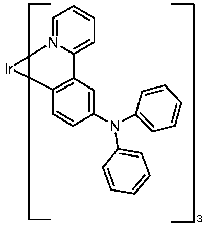 | 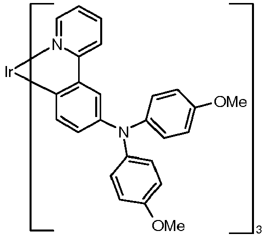 | 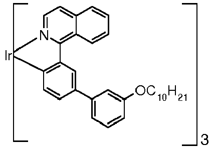 |
| (T-16) | (T-17) | (T-18) |
| 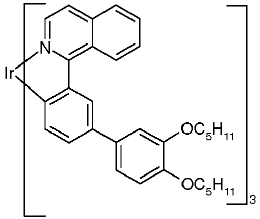 | 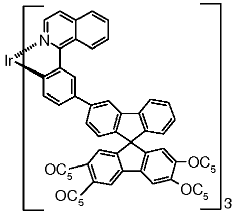 | 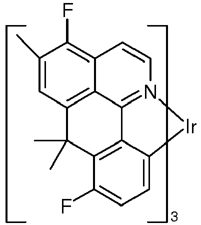 |
| (T-19) | (T-20) | (T-21) |
| 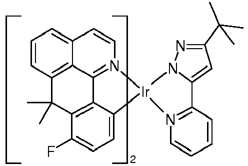 | 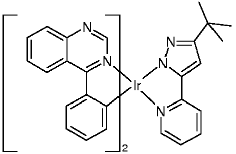 | 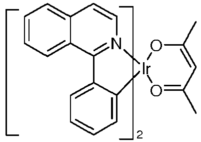 |
| (T-22) | (T-23) | (T-24) |
| 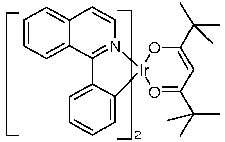 | 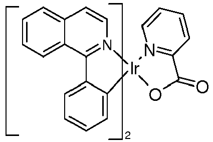 | 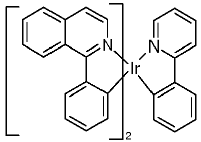 |
| (T-25) | (T-26) | (T-27) |
| 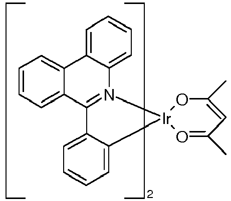 | 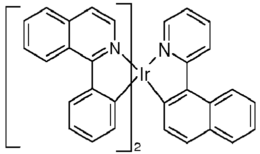 | 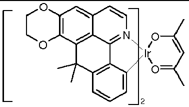 |
| (T-28) | (T-29) | (T-30) |
| 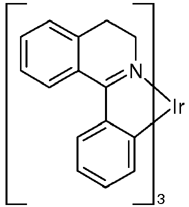 | 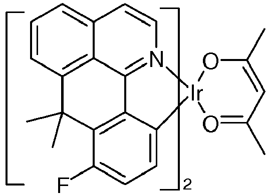 | 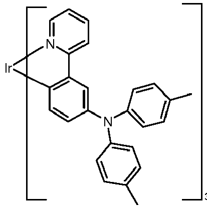 |
| (T-31) | (T-32) | (T-33) |
| 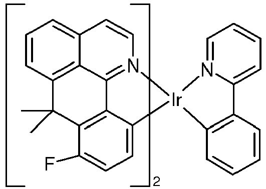 | 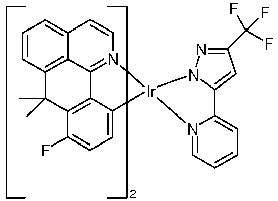 | 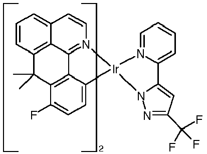 |
| (T-34) | (T-35) | (T-36) |
| 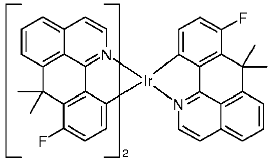 | 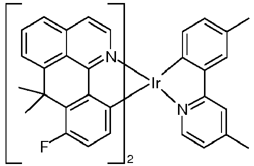 | 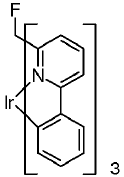 |
| (T-37) | (T-38) | (T-39) |
| 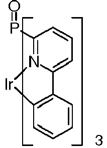 | 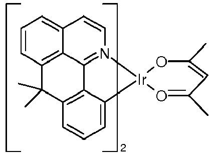 | 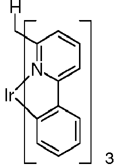 |
| (T-40) | (T-41) | (T-42) |
| 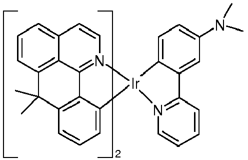 | 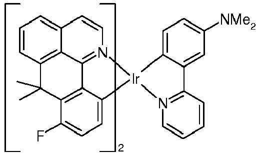 | 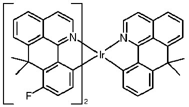 |
| (T-43) | (T-44) | (T-45) |
| 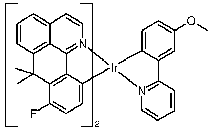 | 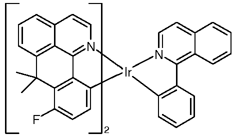 | 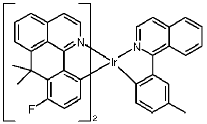 |
| (T-46) | (T-47) | (T-48) |
| 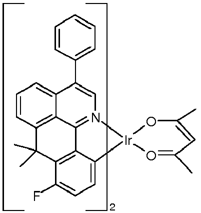 | 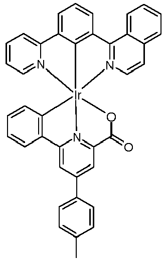 | 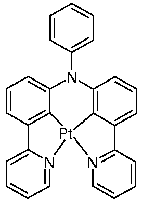 |
| (T-49) | (T-50) | (T-51) |
| 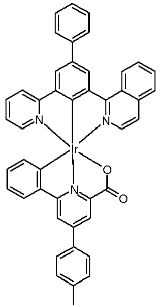 | 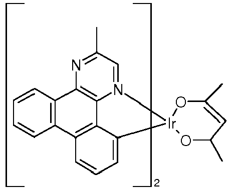 | 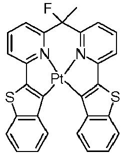 |
| (T-52) | (T-53) | (T-54) |
| 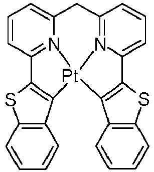 | 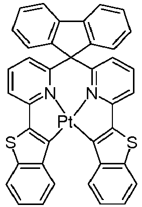 | 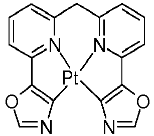 |
| (T-55) | (T-56) | (T-57) |
| 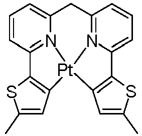 | 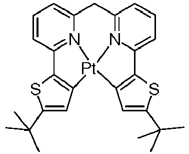 | 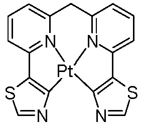 |
| (T-58) | (T-59) | (T-60) |
| 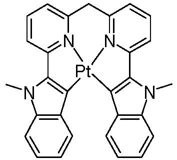 | 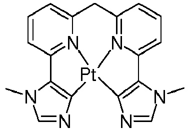 | 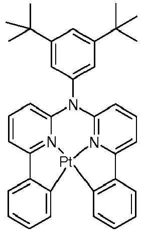 |
| (T-61) | (T-62) | (T-63) |
| 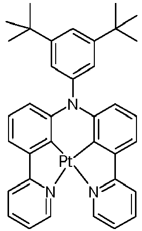 | 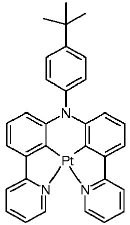 | 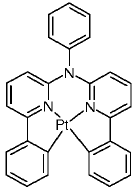 |
| (T-64) | (T-65) | (T-66) |
| 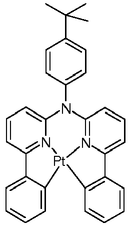 | 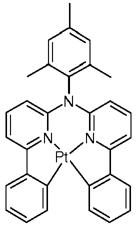 | 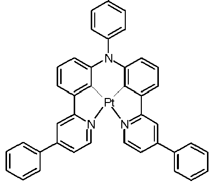 |
| (T-67) | (T-68) | (T-69) |
| 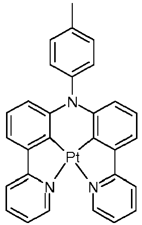 | 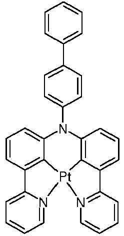 | 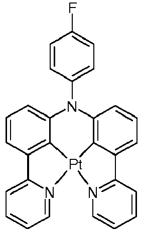 |
| (T-70) | (T-71) | (T-72) |
| 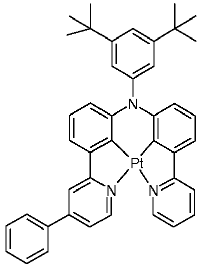 | 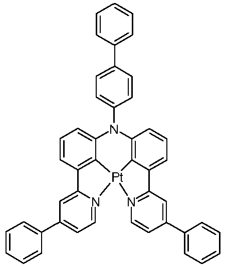 | 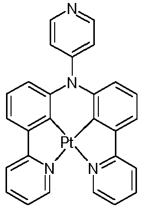 |
| (T-73) | (T-74) | (T-75) |
| 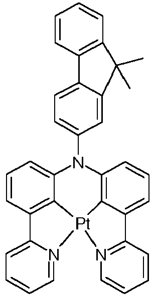 | 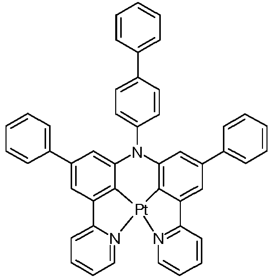 | 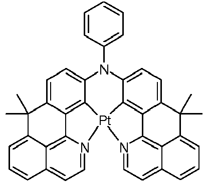 |
| (T-76) | (T-77) | (T-78) |
| 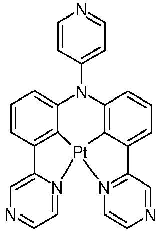 | 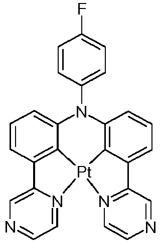 | 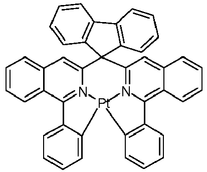 |
| (T-79) | (T-80) | (T-81) |
| 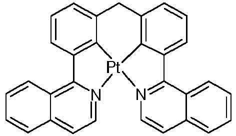 | 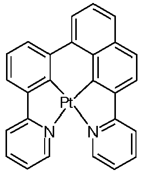 | 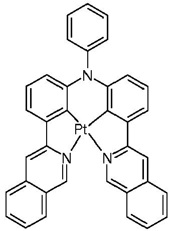 |
| (T-82) | (T-83) | (T-84) |
| 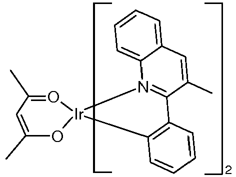 | 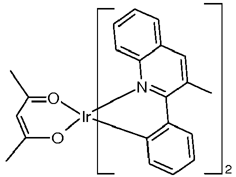 | 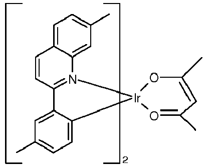 |
| (T-85) | (T-86) | (T-87) |
| 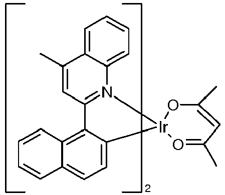 | 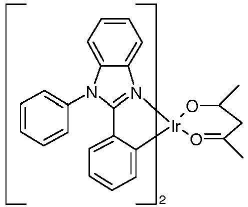 | 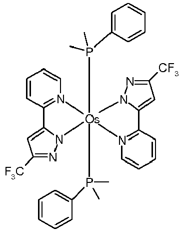 |
| (T-88) | (T-89) | (T-90) |
| 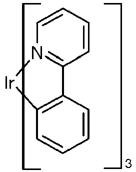 | 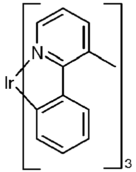 | 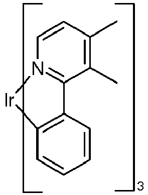 |
| (T-91) | (T-92) | (T-93) |
| 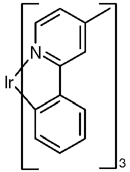 | 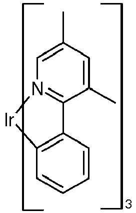 | 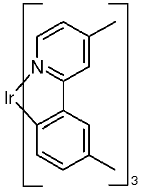 |
| (T-94) | (T-95) | (T-96) |
| 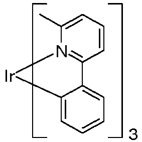 | 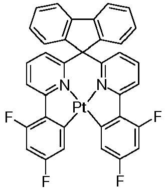 | 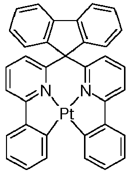 |
| (T-97) | (T-98) | (T-99) |
| 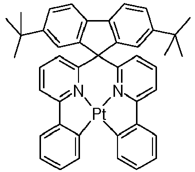 | 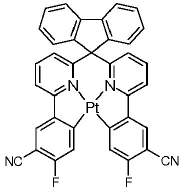 | 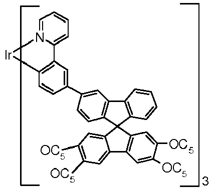 |
| (T-100) | (T-101) | (T-102) |
| 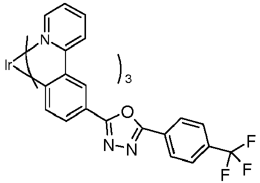 | 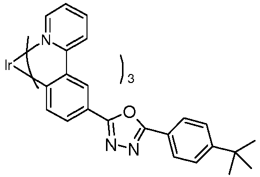 | 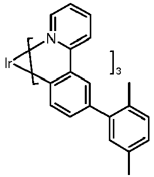 |
| (T-103) | (T-104) | (T-105) |
| 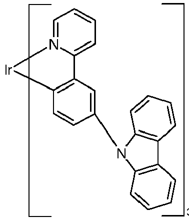 | 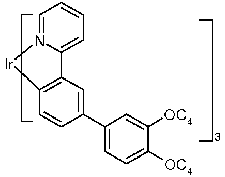 | 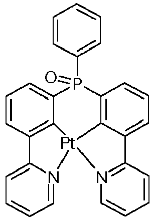 |
| (T-106) | (T-107) | (T-108) |
| 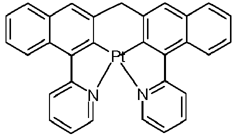 | 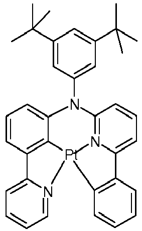 | 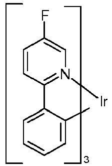 |
| (T-109) | (T-110) | (T-111) |
| 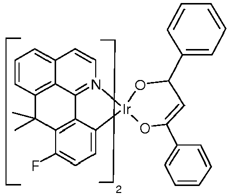 | 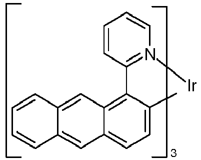 | 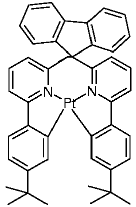 |
| (T-112) | (T-113) | (T-114) |
| 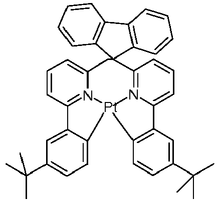 | 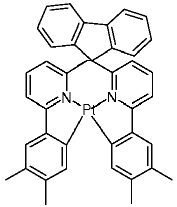 | 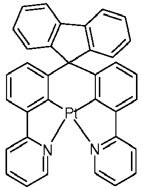 |
| (T-115) | (T-116) | (T-117) |
| 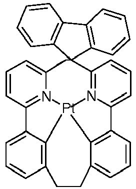 | 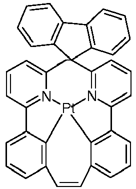 | 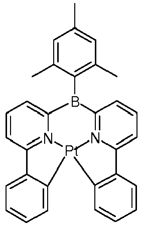 |
| (T-118) | (T-119) | (T-120) |
| 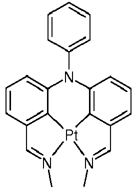 | 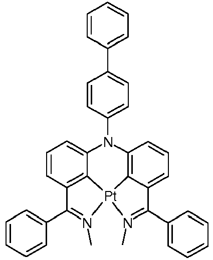 | 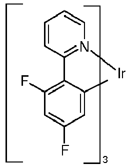 |
| (T-121) | (T-122) | (T-123) |
| 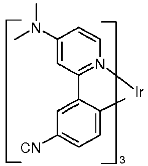 | 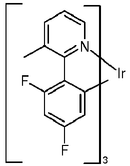 | 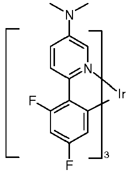 |
| (T-124) | (T-125) | (T-126) |
| 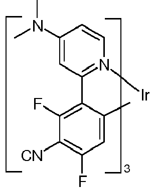 | 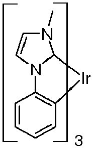 | 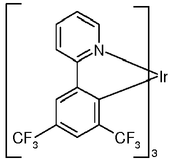 |
| (T-127) | (T-128) | (T-129) |
| 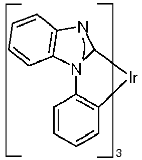 | 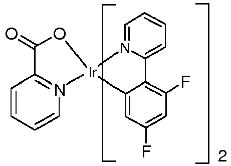 | 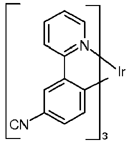 |
| (T-130) | (T-131) | (T-132) |
| 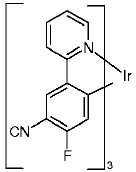 | 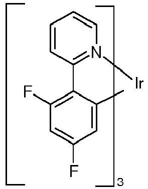 | 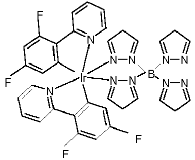 |
| (T-133) | (T-134) | (T-135) |
| 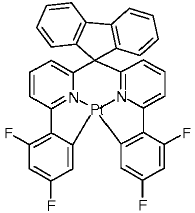 | 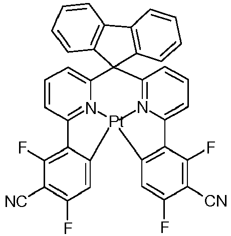 | 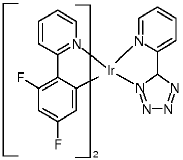 |
| (T-136) | (T-137) | (T-138) |
| 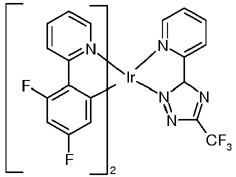 | 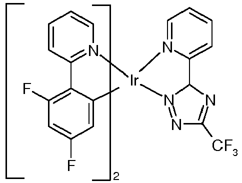 | |
| (T-139) | (T-140) | |

Die erfindungsgemäße Formulierung umfasst ferner mindestens ein Lösungsmittel. Die Formulierung eignet sich hervorragend zum Erzeugen von Schichten aus Lösung.

Geeignete und bevorzugte Lösungsmittel sind beispielsweise Toluol, Anisol, Xylole, Methylbenzoat, Dimethylanisole, Trimethylbenzole, Tetralin, Veratrole, Tetrahydrofuran, Chlorbenzol und Dichlorbenzol sowie Gemische derselben.

Die erfindungsgemäße Formulierung eignet sich für die Herstellung von organischen Elektrolumineszenzvorrichtungen (OLEDs, PLEDs), insbesondere für eine Lumineszenzschicht solcher Vorrichtungen. Insbesondere liegen die einzelnen Komponenten der Formulierung als homogene Lösung vor, wodurch sich die Formulierung besonders gut zu einer Schicht verarbeiten läßt.

Ein weiterer Gegenstand der vorliegenden Erfindung ist daher die Verwendung der erfindungsgemäßen Formulierung zur Herstellung von organischen Elektrolumineszenzvorrichtungen.

Nochmals ein weiterer Gegenstand der vorliegenden Erfindung sind organische Elektrolumineszenzvorrichtungen, welche unter Verwendung der erfindungsgemäßen Formulierung hergestellt werden, enthaltend Anode, Kathode und mindestens eine emittierende Schicht, dadurch gekennzeichnet, dass mindestens eine emittierende Schicht aus einer erfindungsgemäßen Mischung erhalten wird.

Außer Kathode, Anode und der mindestens einen emittierenden Schicht, kann die organische Elektrolumineszenzvorrichtung noch weitere Schichten enthalten. Diese sind beispielsweise ausgewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) und/oder organischen oder anorganischen p/n-Übergängen. Außerdem können Interlayers vorhanden sein, welche beispielsweise die Ladungsbalance im Device steuern. Insbesondere können solche Interlayers als Zwischenschicht zwischen zwei emittierenden Schichten sinnvoll sein, insbesondere als Zwischenschicht zwischen einer fluoreszierenden und einer phosphoreszierenden Schicht. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede der oben genannten Schichten vorhanden sein muss und die Wahl der Schichten immer von den verwendeten Verbindungen abhängt. Die Verwendung derartiger Schichten ist dem Fachmann bekannt, und er kann hierfür ohne erfinderisches Zutun alle für derartige Schichten bekannten Materialien gemäß dem Stand der Technik verwenden.

Weiterhin ist es möglich, mehr als eine emittierende Schicht zu verwenden, beispielsweise zwei oder drei emittierende Schichten, wobei diese vorzugsweise unterschiedliche Emissionsfarben aufweisen. In einer besonders bevorzugten Ausführungsform der vorliegenden Erfindung handelt es sich um eine weiß emittierende organische Elektrolumineszenzvorrichtung. Diese ist dadurch gekennzeichnet, dass sie Licht mit CIE-Farbkoordinaten im Bereich von 0,28/0,29 bis 0,45/0,41 emittiert. Der allgemeine Aufbau einer derartigen weiß emittierenden Elektrolumineszenzvorrichtung wird beispielsweise in der WO 05/011013 offenbart.

Als Kathode der erfindungsgemäßen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z.B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z.B. Ag, wobei dann in der Regel Kombinationen der Metalle, wie beispielsweise Ca/Ag oder Ba/Ag verwendet werden. Ebenso bevorzugt sind Metalllegierungen, insbesondere Legierungen aus einem Alkalimetall oder Erdalkalimetall und Silber, besonders bevorzugt eine Legierung aus Mg und Ag. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z.B. LiF, Li₂O, CsF, Cs₂CO₃, BaF₂, MgO, NaF, etc.). Die Schichtdicke dieser Schicht beträgt vorzugsweise zwischen 0,5 und 5 nm.

Als Anode der erfindungsgemäßen Elektrolumineszenzvorrichtung sind Materialien mit hoher Austrittsarbeit bevorzugt. Vorzugsweise weist die Anode eine Austrittsarbeit größer 4,5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z.B. Al/Ni/NiOₓ, Al/PtOₓ) bevorzugt sein. Dabei muss mindestens eine der Elektroden transparent sein, um die Auskopplung von Licht zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, wie z.B. PEDOT oder PANI.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Es können generell alle weiteren Materialien, wie sie gemäß dem Stand der Technik in organischen Elektrolumineszenzvorrichtungen eingesetzt werden, in Kombination mit der emittierenden Schicht, welche die erfindungsgemäße Mischung enthält, eingesetzt werden.

Weiterhin bevorzugt ist eine organische Elektrolumineszenzvorrichtung, die dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, vorzugsweise kleiner 10⁻⁶ mbar aufgedampft. Es sei jedoch angemerkt, dass der Anfangsdruck auch noch geringer sein kann, beispielsweise kleiner 10⁻⁷ mbar.

Bevorzugt ist ebenfalls eine organische Elektrolumineszenzvorrichtung, die dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z.B. M. S. Arnold et al., Appl. Phys. Lett. 2008, 92, 053301).

Besonders bevorzugt ist eine organische Elektrolumineszenzvorrichtung, die dadurch gekennzeichnet ist, dass eine oder mehrere Schichten aus Lösung, wie z.B. durch Spincoating, oder mit einem beliebigen Druckverfahren, wie z.B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, wie sie in der erfindungsgemäßen Mischung vorhanden sind.

Die organische Elektrolumineszenzvorrichtung kann auch als Hybridsystem hergestellt werden, indem eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf die erfindungsgemäßen organischen Elektrolumineszenzvorrichtungen angewandt werden.

Die erfindungsgemäßen organischen Elektrolumineszenzvorrichtungen weisen die folgenden überraschenden Vorteile gegenüber dem Stand der Technik auf:
1. Die erfindungsgemäße organische Elektrolumineszenzvorrichtung weist eine sehr hohe Effizienz auf. Dabei ist die Effizienz besser als bei Verwendung eines elektronentransportierenden Matrixmaterials in Kombination mit einem lochtransportierenden Matrixmaterial gemäß dem Stand der Technik.
2. Die erfindungsgemäße organische Elektrolumineszenzvorrichtung weist gleichzeitig eine verbesserte Lebensdauer auf. Dabei ist die Lebensdauer höher als bei Verwendung eines elektronentransportierenden Matrixmaterials in Kombination mit einem lochtransportierenden Matrixmaterial gemäß dem Stand der Technik.

Die Erfindung wird durch die nachfolgenden Beispiele näher beschrieben, ohne sie dadurch einschränken zu wollen. Der Fachmann kann, ohne erfinderisch tätig zu werden, weitere erfindungsgemäße organische Elektrolumineszenzvorrichtungen herstellen.

### Allgemeine Methoden:

### Bestimmung von HOMO, LUMO und Energielücke aus Cyclovoltammetrie und Absortionsspektrum

Die Bestimmung der HOMO- und LUMO-Werte sowie der Energielücke gemäß der vorliegenden Anmeldung erfolgt nach den im Folgenden beschriebenen allgemeinen Verfahren.

Der HOMO-Wert ergibt sich aus dem Oxidationspotential, das mittels Cyclovoltammetrie (CV) bei Raumtemperatur gemessen wird. Als Messgerät hierzu wird ein ECO Autolab System mit Metrohm 663 VA Stand verwendet. Die Arbeitselektrode ist eine Goldelektrode, die Referenzelektrode Ag/AgCl, der Zwischenelektrolyt KCl (3 mol/l) und die Hilfselektrode Platin.

Für die Messung wird zunächst eine 0,11 M Leitsalzlösung aus Tetrabutylammoniumhexafluorophosphat (NH₄PF₆) in Dichlormethan hergestellt, in die Messzelle eingefüllt und 5 Minuten entgast. Anschließend werden zwei Messzyklen mit folgenden Parametern durchgeführt:
Messtechnik: CV
Initial purge time: 300 s
Cleaning Potential: -1 V
Cleaning Time: 10 s
Deposition Potential: -0,2 V
Deposition Time: 10 s
Start Potential: -0,2 V
End Potential: 1,6 V
Voltage Step: 6 mV
Sweep Rate: 50 mV/s

Anschließend wird die Leitsalzlösung mit 1 ml der Probenlösung (10 mg der zu messenden Substanz in 1 ml Dichlormethan) versetzt und erneut 5 Minuten entgast. Anschließend werden fünf weitere Messzyklen gefahren, von denen die letzten 3 zur Auswertung aufgezeichnet werden. Dabei werden dieselben Parameter eingestellt, wie oben beschrieben.

Anschließend wird die Lösung mit 0,1 ml Ferrocenlösung (100 mg Ferrocen in 1 ml Dichlormethan) versetzt, 1 Minute entgast, und ein Messzyklus mit folgenden Parametern durchgeführt:
Messtechnik: CV
Initial purge time: 60 s
Cleaning Potential: -1 V
Cleaning Time: 10 s
Deposition Potential: -0,2 V
Deposition Time: 10 s
Start Potential: -0,2 V
End Potential: 1,6 V
Voltage Step: 6 mV
Sweep Rate: 50 mV/s

Zur Auswertung wird für die Probenlösung und die mit Ferrocenlösung versetzte Lösung jeweils der Mittelwert aus den Spannungen des ersten Oxidationsmaximums aus den Hinkurven und des zugehörigen Reduktionsmaximums aus den Rückkurven genommen (V_{P} und V_{F}), wobei als Spannung jeweils die Spannung gegen Ferrocen verwendet wird. Der HOMO-Wert der zu untersuchenden Substanz E_{HOMO} ergibt sich als E_{HOMO} = -[e·(V_{P} - V_{F}) + 4,8 eV], wobei e die Elementarladung darstellt.

Es ist zu beachten, dass im Einzelfall gegebenenfalls sinnvolle Abwandlungen der Messmethode vorgenommen werden müssen, z.B. wenn sich die zu untersuchende Substanz in Dichlormethan nicht lösen lässt oder wenn es zur Zersetzung der Substanz während der Messung kommt. Sollte eine sinnvolle Messung mittels CV mit der oben genannten Methode nicht möglich sein, wird die HOMO-Energie über Photoelektronen-Spektroskopie mittels Model AC-2 Photoelectron Spectrometer von Riken Keiki Co. Ltd. (http://www.rikenkeiki.com/pages/AC2.htm) bestimmt, wobei zu beachten ist, dass die erhaltenen Werte dabei typischerweise um 0,3 eV tiefer liegen, als die mit CV gemessenen. Unter dem HOMO-Wert gemäß der vorliegenden Anmeldung ist dann der Wert aus Riken AC2 + 0,3 eV zu verstehen.

Weiterhin lassen sich sowohl mit der beschriebenen CV Methode wie auch mit der beschriebenen Photoelektronen-Spektroskopie HOMO-Werte, die tiefer als -6 eV liegen, nicht verlässlich messen. In diesem Fall werden die HOMO-Werte aus quantenchemischer Rechnung mittels Dichtefunktional-Theorie (DFT) ermittelt. Dies erfolgt über die kommerziell erhältliche Software Gaussian 03W (Gaussian Inc.) mit der Methode B3PW91/6-31 G(d). Die Normierung der berechneten Werte auf CV-Werte wird über einen Abgleich mit aus CV messbaren Materialien erreicht. Hierzu werden die HOMO-Werte einer Reihe von Materialien mit der CV Methode gemessen und ebenso berechnet. Die berechneten Werte werden dann mittels der gemessenen kalibriert, dieser Kalibrierfaktor wird für alle weiteren Berechnungen verwendet. Auf diese Weise lassen sich HOMO-Werte berechnen, die sehr gut denen entsprechen, die man über CV messen würde. Sollte sich der HOMO-Wert einer bestimmten Substanz nicht über CV oder Riken AC2 wie oben beschrieben messen lassen, ist unter dem HOMO-Wert gemäß der vorliegenden Anmeldung daher der Wert zu verstehen, der entsprechend der Beschreibung durch eine auf CV kalibrierte DFT-Rechnung, wie oben beschrieben, erhalten wird. Beispiele für auf diese Weise berechnetete Werte einiger gängiger organischer Materialien sind: NPB = (HOMO -5,16 eV, LUMO -2,28 eV); TCTA (HOMO -5,33 eV, LUMO -2,20 eV); TPBI (HOMO -6,26 eV, LUMO -2,48 eV). Diese Werte können für die Kalibrierung der Rechenmethode verwendet werden.

Die Energielücke wird bestimmt aus der Absorptionskante des Absorptionsspektrums gemessen an einem Film mit Schichtdicke 50 nm. Die Absorptionskante ist dabei definiert als die Wellenlänge, die man erhält, wenn man im Absorptionsspektrum an die langwelligste abfallende Flanke an ihrer steilsten Stelle eine Gerade anfittet und den Wert ermittelt, bei dem diese Gerade die Wellenlängeachse, d.h. den Absorptionswert = 0, schneidet.

Der LUMO Wert wird durch Addition der Energielücke auf den oben beschriebenen HOMO-Wert erhalten.

### Ausführungsbeispiele:

### Beispiele 1 bis 45: Herstellung von PLEDs

Die Strukturen des Emitters E1, der Ketone K-1 bis K-12 (Referenz-Materialien), der erfindungsgemäßen Triazine T-1 bis T-4 und der erfindungs-gemäßen Carbazole C-1 bis C-6 sind im Folgenden abgebildet.

### Struktur des Emitters E1

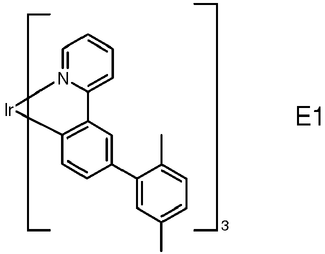

**Strukturen der Keton-Verbindungen (Referenz-Materialien)**

| | |
|---|---|
| 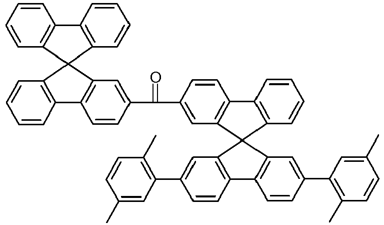 | 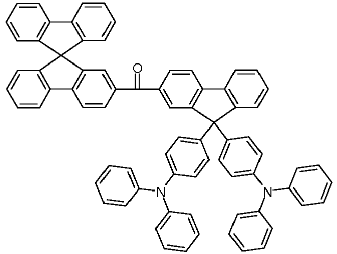 |
| K-1 | K-2 |
| 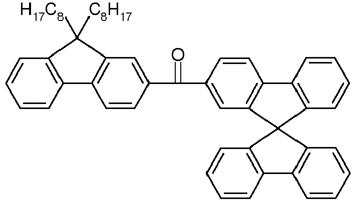 | 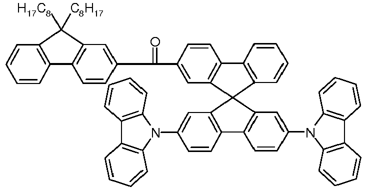 |
| K-3 | K-4 |
| 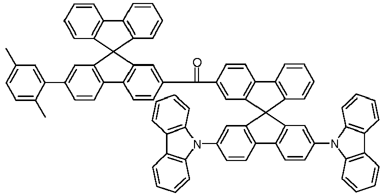 | 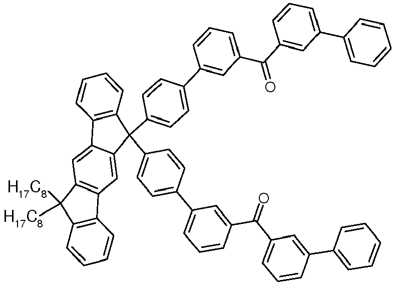 |
| K-5 | K-6 |
| 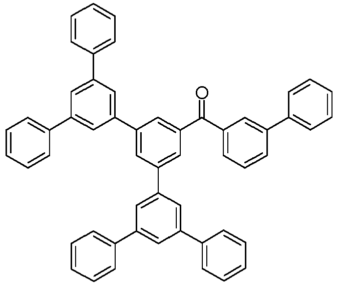 | 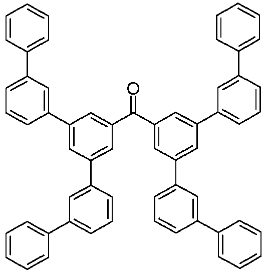 |
| K-7 | K-8 |
| 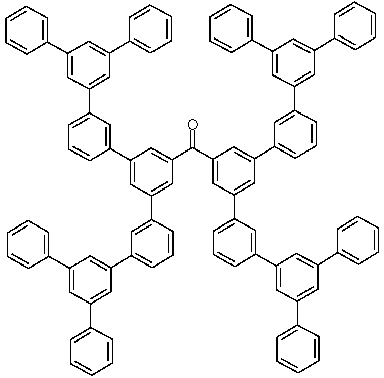 | 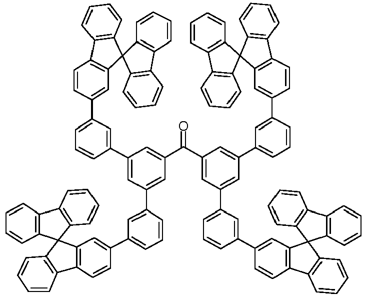 |
| K-9 | K-10 |
| 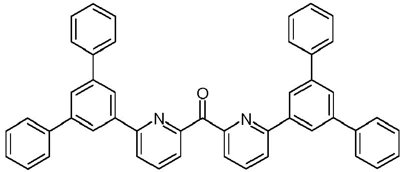 | 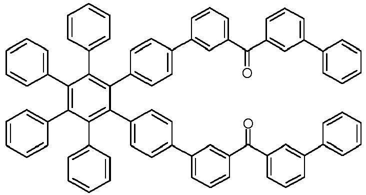 |
| K-11 | K-12 |

**Strukturen der Triazin-Verbindungen**

| | |
|---|---|
| 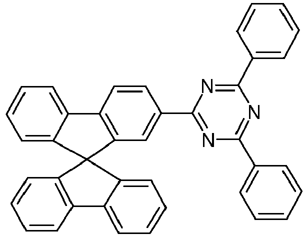 | 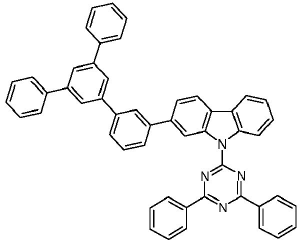 |
| T-1 | T-2 |
| 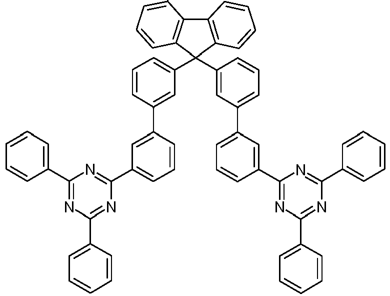 | 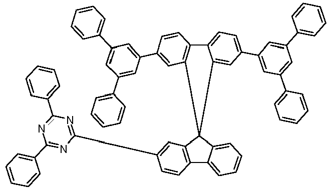 |
| T-3 | T-4 |

**Strukturen der Carbazol-Verbindungen**

| | |
|---|---|
| 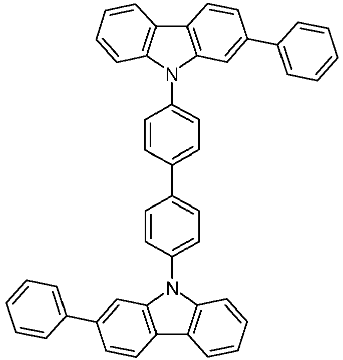 | 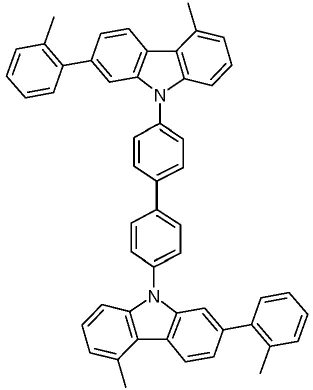 |
| C-1 | C-2 |
| 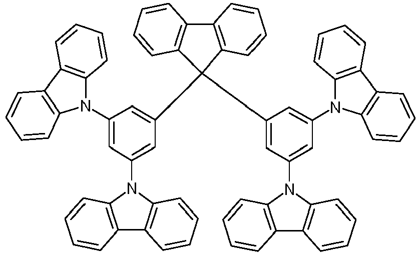 | 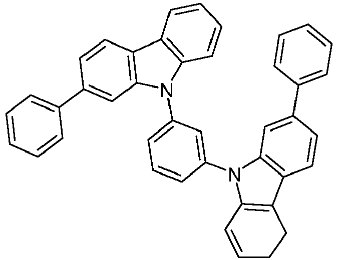 |
| C-3 | C-4 |
| 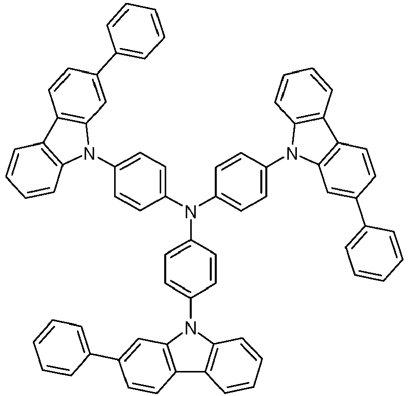 | 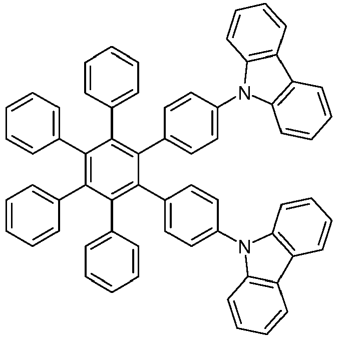 |
| C-5 | C-6 |

Die Materialien, die in den erfindungsgemäßen Formulierungen verwendet werden, führen dort zu wesentlich einfacheren Devices mit guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887 A2). Im vorliegenden Fall werden die erfindungsgemäßen Verbindungen in Toluol bzw. Chlorbenzol gelöst. Die in den hier genannten Beispielen eingesetzte Konzentration beträgt 20 Gew.-% des Emitters, 40 Gew.-% der Verbindungen K-1 bis K-12 (Referenzmaterialien) bzw. der Verbindungen T-1 bis T-4 und 40 Gew.-% der Verbindungen C-1 bis C-6. Der typische Feststoffgehalt solcher Lösungen liegt zwischen 16 und 25 g/l, wenn, wie hier, die für eine Device typische Schichtdicke von 80 nm mittels Spincoating erzielt werden soll.

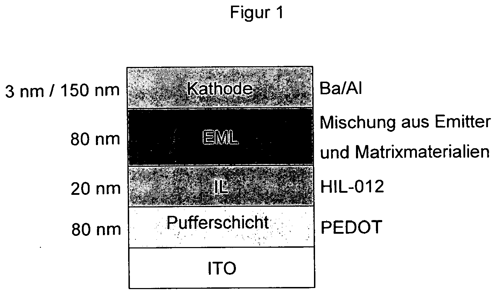

Fig. 1 zeigt den typischen Aufbau einer solchen Device. In der EML liegen die Matrixmaterialien sowie der Emitter in Form einer amorphen Schicht vor. Strukturierte ITO-Substrate und das Material für die sogenannte Pufferschicht (PEDOT, eigentlich PEDOT:PSS) sind käuflich erhältlich (ITO unter anderem von Technoprint, PEDOT:PPS als wässrige Dispersion Clevios P von H.C. Starck). Die verwendete Interlayer dient der Lochinjektion. Es wird HIL-012 von Merck verwendet. Die Emissionsschicht wird in einer Inertgasatmosphäre, im vorliegenden Fall Argon, aufgeschleudert und 10 Minuten bei 160°C bzw. 180°C ausgeheizt. Zuletzt wird eine Kathode aus Barium und Aluminium im Vakuum aufgedampft. Zwischen EML und Kathode können auch weitere Schichten (z.B. HBL und ETL) mittels Bedampfung aufgebracht werden. Auch kann die Interlayer durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Die lösungsprozessierten Devices werden standardmäßig charakterisiert, die genannten OLED-Beispiele wurden nicht optimiert.

In Tabelle 1 werden die Deviceergebnisse ohne die erfindungsgemäßen Verbindungen C-1 bis C-6 mit denen, die durch eine gemischte Schicht mit den Referenz-Materialien K-1 bis K-12 oder den erfindungsgemäßen Materialien T-1 bis T-4 erhalten wurden, verglichen.

| Tabelle 1: Ergebnisse in der Devicekonfiguration der Figur 1 | | | | | |
|---|---|---|---|---|---|
| Bsp. | EML 80 nm | Max. Eff. [cd/A] | Spannung [V] bei 100 cd/m² | CIE (x, y) | Lebensdauer [h], Anfangshelligkeit 1000 cd/m² |
| Vergl. | K-1 : E-1 | 22 | 4,2 | 0,35/0,61 | 2700 |
| Ref. | K-1 : C-1 : E-1 | 30 | 4,3 | 0,36/0,61 | 8500 |
| Ref. | K-1 : C-2 : E-1 | 27 | 4,1 | 0,35/0,61 | 4100 |
| Ref. | K-1 : C-3 : E-1 | 29 | 4,1 | 0,35/0,61 | 4000 |
| Ref. | K-1 : C-4 : E-1 | 28 | 4,1 | 0,35/0,61 | 5700 |
| Ref. | K-1 : C-5 : E-1 | 32 | 4,1 | 0,34/0,62 | 4500 |
| Ref. | K-1 : C-6 : E-1 | 27 | 4,5 | 0,34/0,62 | 3950 |
| Vergl. | K-2 : E-1 | 26 | 4,5 | 0,35/0,61 | 1100 |
| Ref. | K-2 : C-1 : E-1 | 29 | 4,2 | 0,36/0,61 | 8500 |
| Ref. | K-2 : C-2 : E-1 | 31 | 4,2 | 0,35/0,61 | 5200 |
| Ref. | K-2 : C-3 : E-1 | 28 | 4,3 | 0,35/0,61 | 3200 |
| Ref. | K-2 : C-4 : E-1 | 29 | 4,2 | 0,35/0,61 | 8600 |
| Ref. | K-2 : C-5 : E-1 | 30 | 4,1 | 0,34/0,62 | 4500 |
| Ref. | K-2 : C-6 : E-1 | 30 | 4,1 | 0,34/0,62 | 6800 |
| Vergl. | K-3 : E-1 | 9 | 4,9 | 0,32/0,63 | 600 |
| Ref. | K-3 : C-1 : E-1 | 20 | 4,6 | 0,32/0,63 | 2180 |
| Ref. | K-3 : C-2 : E-1 | 18 | 4,5 | 0,35/0,61 | 3000 |
| Ref. | K-3 : C-3 : E-1 | 15 | 4,6 | 0,35/0,61 | 2200 |
| Ref. | K-3 : C-4 : E-1 | 23 | 4,4 | 0,35/0,61 | 3600 |
| Ref. | K-3 : C-5 : E-1 | 19 | 4,4 | 0,34/0,62 | 4500 |
| Ref. | K-3 : C-6 : E-1 | 21 | 4,5 | 0,34/0,62 | 5900 |
| Vergl. | K-4 : E-1 | 20 | 4,0 | 0,34/0,62 | 1425 |
| Ref. | K-4 : C-1 : E-1 | 28 | 4,0 | 0,34/0,62 | 4500 |
| Ref. | K-4 : C-2 : E-1 | 25 | 4,1 | 0,35/0,61 | 5100 |
| Ref. | K-4 : C-3 : E-1 | 30 | 4,1 | 0,35/0,61 | 6300 |
| Ref. | K-4 : C-4 : E-1 | 27 | 4,1 | 0,35/0,61 | 4300 |
| Ref. | K-4 : C-5 : E-1 | 26 | 4,1 | 0,34/0,62 | 4600 |
| Ref. | K-4 : C-6 : E-1 | 24 | 4,0 | 0,34/0,62 | 3900 |
| Vergl. | K-5 : E-1 | 24 | 4,0 | 0,36/0,60 | 1800 |
| Ref. | K-5 : C-1 : E-1 | 29 | 4,0 | 0,34/0,62 | 3300 |
| Ref. | K-5 : C-2 : E-1 | 28 | 4,0 | 0,35/0,61 | 4500 |
| Ref. | K-5 : C-3 : E-1 | 29 | 3,9 | 0,35/0,61 | 4200 |
| Ref. | K-5 : C-4 : E-1 | 29 | 3,9 | 0,35/0,61 | 3600 |
| Ref. | K-5 : C-5 : E-1 | 30 | 4,0 | 0,34/0,62 | 4800 |
| Ref. | K-5 : C-6 : E-1 | 28 | 4,1 | 0,34/0,62 | 4900 |
| Vergl. | K-6 : E-1 | 20 | 7,5 | 0,33/0,62 | 1500 |
| Ref. | K-6 : C-1 : E-1 | 27 | 6,0 | 0,33/0,62 | 8000 |
| Ref. | K-6 : C-2 : E-1 | 27 | 4,7 | 0,34/0,62 | 7200 |
| Ref. | K-6 : C-3 : E-1 | 27 | 4,5 | 0,35/0,61 | 8100 |
| Ref. | K-6 : C-4 : E-1 | 29 | 4,6 | 0,35/0,61 | 5600 |
| Ref. | K-6 : C-5 : E-1 | 28 | 4,3 | 0,35/0,61 | 3700 |
| Ref. | K-6 : C-6 : E-1 | 32 | 4,5 | 0,34/0,62 | 6300 |
| Vergl. | K-7 : E-1 | 21 | 5,7 | 0,33/0,63 | 1200 |
| Ref. | K-7 : C-1 : E-1 | 29 | 4,4 | 0,33/0,63 | 1600 |
| Ref. | K-7 : C-2 : E-1 | 25 | 4,5 | 0,35/0,61 | 3100 |
| Ref. | K-7 : C-3 : E-1 | 28 | 4,3 | 0,35/0,61 | 5200 |
| Ref. | K-7 : C-4 : E-1 | 27 | 4,4 | 0,35/0,61 | 5300 |
| Ref. | K-7 : C-5 : E-1 | 28 | 4,5 | 0,34/0,62 | 3900 |
| Ref. | K-7 : C-6 : E-1 | 30 | 4,4 | 0,34/0,62 | 5100 |
| Vergl. | K-8 : E-1 | 27 | 5,0 | 0,35/0,61 | 3200 |
| Ref. | K-8 : C-1 : E-1 | 33 | 4,7 | 0,34/0,62 | 10000 |
| Ref. | K-8 : C-2 : E-1 | 31 | 4,7 | 0,34/0,61 | 7000 |
| Ref. | K-8 : C-3 : E-1 | 31 | 4,7 | 0,34/0,61 | 7000 |
| Ref. | K-8 : C-4 : E-1 | 33 | 4,5 | 0,33/0,63 | 9000 |
| Ref. | K-8 : C-5 : E-1 | 32 | 4,9 | 0,33/0,62 | 5900 |
| Ref. | K-7 : C-6 : E-1 | 32 | 4,6 | 0,34/0,62 | 6200 |
| Vergl. | K-9 : E-1 | 26 | 6,3 | 0,34/0,62 | 1010 |
| Ref. | K-9 : C-1 : E-1 | 35 | 5,2 | 0,34/0,62 | 3000 |
| Ref. | K-9 : C-2 : E-1 | 33 | 5,3 | 0,34/0,62 | 3100 |
| Ref. | K-9 : C-3 : E-1 | 31 | 5,2 | 0,34/0,62 | 4000 |
| Ref. | K-9 : C-4 : E-1 | 32 | 5,1 | 0,34/0,62 | 4100 |
| Ref. | K-9 : C-5 : E-1 | 30 | 5,2 | 0,34/0,62 | 3200 |
| Ref. | K-9 : C-6 : E-1 | 31 | 5,3 | 0,34/0,62 | 3400 |
| Vergl. | K-10 : E-1 | 26 | 6,0 | 0,32/0,63 | 1400 |
| Ref. | K-10 : C-1 : E-1 | 30 | 5,5 | 0,32/0,63 | 5600 |
| Ref. | K-10 : C-2 : E-1 | 31 | 5,3 | 0,34/0,62 | 2100 |
| Ref. | K-10 : C-3 : E-1 | 33 | 5,4 | 0,34/0,62 | 4500 |
| Ref. | K-10 : C-4 : E-1 | 32 | 5,5 | 0,34/0,62 | 6500 |
| Ref. | K-10 : C-5 : E-1 | 30 | 5,3 | 0,34/0,62 | 4300 |
| Ref. | K-10 : C-6 : E-1 | 29 | 5,5 | 0,34/0,62 | 3400 |
| Vergl. | K-11 : E-1 | 30 | 5,5 | 0,34/0,62 | 7000 |
| Ref. | K-11 : C-1 : E-1 | 35 | 5,1 | 0,35/0,62 | 9000 |
| Ref. | K-11 : C-2 : E-1 | 34 | 4,9 | 0,35/0,62 | 8500 |
| Ref. | K-11 : C-6 : E-1 | 35 | 5,0 | 0,35/0,62 | 9500 |
| Vergl. | K-12 : E-1 | 25 | 8,3 | 0,34/0,62 | 2200 |
| Ref. | K-12 : C-1 : E-1 | 32 | 6,9 | 0,35/0,62 | 7000 |
| Vergl. | T-1 : E-1 | 14 | 4,2 | 0,36/0,61 | 9200 |
| | T-1 : C-1 : E-1 | 24 | 3,5 | 0,34/0,62 | 10500 |
| | T-1 : C-2 : E-1 | 23 | 3,3 | 0,34/0,62 | 10400 |
| | T-1 : C-3 : E-1 | 19 | 3,3 | 0,33/0,62 | 10200 |
| | T-1 : C-4 : E-1 | 20 | 3,3 | 0,33/0,62 | 10100 |
| | T-1 : C-5 : E-1 | 23 | 3,3 | 0,33/0,62 | 10600 |
| | T-1 : C-6 : E-1 | 21 | 3,3 | 0,33/0,62 | 10400 |
| Vergl. | T-2 : E-1 | 25 | 5,3 | 0,33/0,62 | 5700 |
| | T-2 : C-1 : E-1 | 28 | 5,2 | 0,33/0,63 | 8200 |
| | T-2 : C-2 : E-1 | 27 | 5,1 | 0,34/0,62 | 3993 |
| | T-2 : C-3 : E-1 | 29 | 5,3 | 0,33/0,62 | 8400 |
| | T-2 : C-4 : E-1 | 28 | 5,2 | 0,33/0,62 | 7500 |
| | T-2 : C-5 : E-1 | 27 | 5,3 | 0,33/0,62 | 9000 |
| | T-2 : C-6 : E-1 | 29 | 3,3 | 0,33/0,62 | 10100 |
| Vergl. | T-3 : E-1 | 28 | 5,4 | 0,34/0,62 | 7200 |
| | T-3 : C-1 : E-1 | 33 | 3,5 | 0,34/0,62 | 17800 |
| | T-3 : C-2 : E-1 | 30 | 3,4 | 0,34/0,62 | 3993 |
| | T-3 : C-3 : E-1 | 31 | 3,5 | 0,33/0,62 | 6200 |
| | T-3 : C-4 : E-1 | 32 | 3,5 | 0,33/0,62 | 5300 |
| | T-3 : C-5 : E-1 | 30 | 3,5 | 0,33/0,62 | 8000 |
| | T-3 : C-6 : E-1 | 30 | 3,5 | 0,33/0,62 | 8300 |
| Vergl. | T-4 : E-1 | 25 | 3,9 | 0,33/0,62 | 5900 |
| | T-4 : C-1 : E-1 | 29 | 3,5 | 0,34/0,62 | 6250 |
| | T-4 : C-2 : E-1 | 30 | 3,4 | 0,34/0,62 | 8300 |
| | T-4 : C-3 : E-1 | 28 | 3,6 | 0,33/0,62 | 7500 |
| | T-4 : C-4 : E-1 | 31 | 3,5 | 0,33/0,62 | 5650 |
| | T-4 : C-5 : E-1 | 30 | 3,5 | 0,33/0,62 | 7300 |
| | T-4 : C-6 : E-1 | 28 | 3,6 | 0,33/0,62 | 9600 |

## Patentansprüche

1. Formulierung enthaltend eine Carbazolverbindung, eine Elektronentransportverbindung, eine Triplett-Emitter-Verbindung und mindestens ein Lösungsmittel, wobei die Elektronentransportverbindung eine Triazinverbindung umfasst und wobei die Carbazolverbindung mindestens zwei Carbazolgruppen aufweist, die über ihre N-Atome miteinander verbunden sind.

2. Formulierung nach Anspruch 1, **dadurch gekennzeichnet, dass** die Carbazolverbindung eine Verbindung der Formel (1) ist 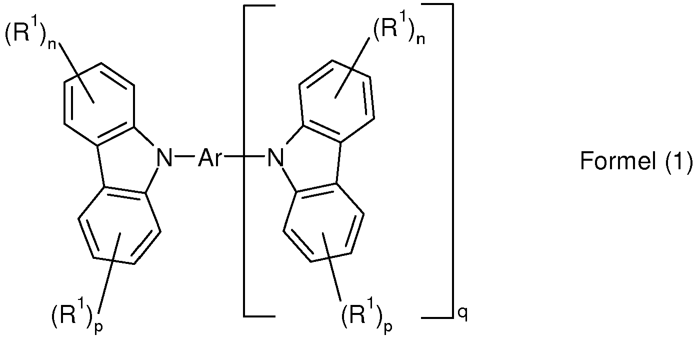 wobei für die verwendeten Symbole und Indices gilt:
Ar ist bei jedem Auftreten ein aromatisches oder heteroaromatisches Ringsystem mit 5 bis 60 aromatischen Ringatomen, welches mit einem oder mehreren Resten R substituiert sein kann;
R, R¹ ist bei jedem Auftreten gleich oder verschieden H, D, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, -CR²=CR²(Ar¹), OSO₂R², eine geradkettige Alkyl-, Alkoxy oder Thioalkoxygruppe mit 1 bis 40 C-Atomen oder eine verzweigte oder cyclische Alkyl-, Alkoxy oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R² substituiert sein kann, oder eine Aryloxy- oder Heteroaryloxygruppe mit 5 bis 60 aromatischen Ringatomen, die mit einem oder mehreren Resten R² substituiert sein kann, oder eine Kombination dieser Systeme; dabei können zwei oder mehrere Substituenten R, R¹ bzw. R und R¹ auch miteinander ein mono- oder polycyclisches, aliphatisches, aromatisches oder heteroaromatisches Ringsystem bilden;
Ar¹ ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
R² ist bei jedem Auftreten gleich oder verschieden H oder ein aliphatischer, aromatischer und/oder heteroaromatischer Kohlenwasserstoffrest mit 1 bis 20 C-Atomen; oder ein aromatisches oder heteroaromatisches Ringsystem mit 5 bis 60 aromatischen Ringatomen, welches mit einem oder mehreren Resten R substituiert sein kann; dabei können zwei oder mehrere Substituenten R² auch miteinander ein mono oder polycyclisches, aliphatisches, aromatisches oder heteroaromatisches Ringsystem bilden;
n ist bei jedem Auftreten gleich oder verschieden 0, 1, 2, 3 oder 4;
p ist bei jedem Auftreten gleich oder verschieden 0, 1, 2, 3 oder 4; und
q ist 1, 2, 3, 4 oder 5.

3. Formulierung nach Anspruch 1, **dadurch gekennzeichnet, dass** die Triazinverbindung eine Verbindung der Formel (7) oder (8) ist, 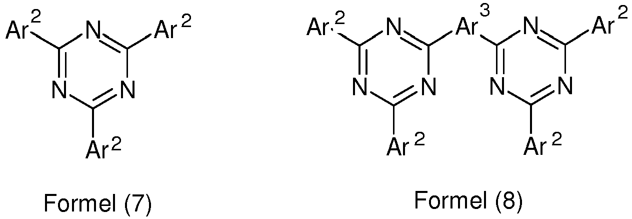 wobei für die weiteren verwendeten Symbole gilt:
Ar¹ ist bei jedem Auftreten gleich oder verschieden ein aromatisches oder heteroaromatisches Ringsystem mit 5 bis 40 aromatischen Ringatomen, das mit einem oder mehreren Resten R² substituiert sein kann;
Ar² ist gleich oder verschieden bei jedem Auftreten ein monovalentes aromatisches oder heteroaromatisches Ringsystem mit 5 bis 60 aromatischen Ringatomen, welches jeweils mit einem oder mehreren Resten R¹ substituiert sein kann;
Ar³ ist ein bivalentes aromatisches oder heteroaromatisches Ring-system mit 5 bis 60 aromatischen Ringatomen, welches mit einem oder mehreren Resten R¹ substituiert sein kann;
R¹ ist bei jedem Auftreten gleich oder verschieden H, D, F, Cl, Br, I, CHO, C(=O)Ar¹, P(=O)(Ar¹)₂, S(=O)Ar¹, S(=O)₂Ar¹, CR²=CR²Aᵣ¹, CN, NO₂, Si(R²)₃, B(OR²)₂, B(R²)₂, B(N(R²)₂)₂, OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches, aromatisches oder heteroaromatisches Ringsystem bilden, und
R² ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches, aliphatisches, aromatisches oder heteroaromatisches Ringsystem bilden.

4. Formulierung nach einem oder mehreren der Ansprüche 1 bis 3, **dadurch gekennzeichnet, dass** die Triplett-Emitter-Verbindung eine Verbindung der Formeln (23) bis (26) ist, 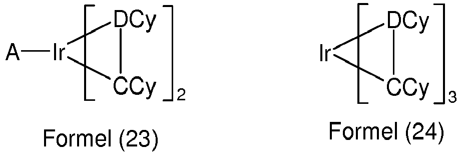 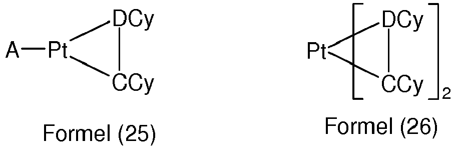 wobei R¹ dieselbe Bedeutung hat, wie in Anspruch 2 beschrieben, und für die weiteren verwendeten Symbole gilt:
DCy ist gleich oder verschieden bei jedem Auftreten eine cyclische Gruppe, die mindestens ein Donoratom, bevorzugt Stickstoff, Kohlenstoff in Form eines Carbens oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
CCy ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann; und
A ist gleich oder verschieden bei jedem Auftreten ein monoanionischer, zweizähnig chelatisierender Ligand, bevorzugt ein Diketonatligand.

5. Verwendung einer Formulierung nach einem oder mehreren der Ansprüche 1 bis 4 zur Herstellung einer organischen Elektrolumineszenzvorrichtung.

6. Organische Elektrolumineszenzvorrichtung umfassend Kathode, Anode und mindestens eine elektrolumineszierende Schicht, **dadurch gekennzeichnet, dass** die elektrolumineszierende Schicht eine Mischung umfassend eine Carbazolverbindung, eine Elektronentransportverbindung und eine Triplett-Emitter-Verbindung enthält, wobei die Elektronentransporteinheit eine Triazinverbindung umfasst und wobei die Carbazolverbindung mindestens zwei Carbazolgruppen aufweist, die über ihre N-Atome miteinander verbunden sind.

7. Organische Elektrolumineszenzvorrichtung nach Anspruch 6, **dadurch gekennzeichnet, dass** die elektrolumineszierende Schicht aus einer Formulierung gemäß einem der Ansprüche 1 bis 5 erhalten wurde.

8. Organische Elektrolumineszenzvorrichtung nach Anspruch 6 oder 7, **dadurch gekennzeichnet, dass** in der Vorrichtung keine Elektroneninjektionsschicht vorhanden ist.

9. Organische Elektrolumineszenzvorrichtung nach einem oder mehreren der Ansprüche 6 bis 8, **dadurch gekennzeichnet, dass** die elektrolumineszierende Schicht aus Lösung aufgebracht ist.

## Claims

1. Formulation comprising a carbazole compound, an electron-transport compound, a triplet emitter compound and at least one solvent, where the electron-transport compound comprises a triazine compound and where the carbazole compound contains at least two carbazole groups which are connected to one another via their N atoms.

2. Formulation according to Claim 1, **characterised in that** the carbazole compound is a compound of the formula (1) 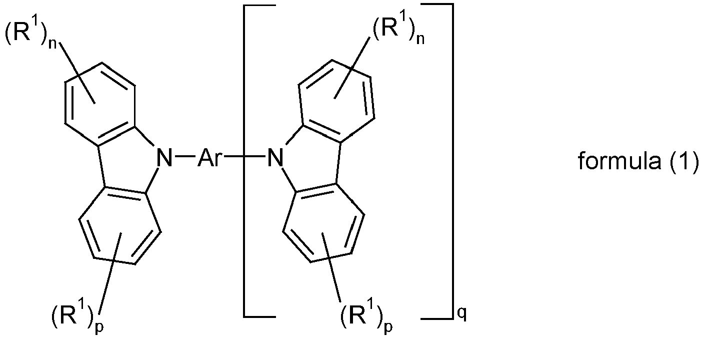 where the following applies to the symbols and indices used:
Ar is on each occurrence an aromatic or heteroaromatic ring system having 5 to 60 aromatic ring atoms, which may be substituted by one or more radicals R;
R, R¹ are on each occurrence, identically or differently, H, D, F, Cl, Br, I, N(Ar¹)₂, CN, NO₂, Si(R²)₃, B(OR²)₂, C(=O)Ar¹, P(=O)(Ar¹)₂, S(=O)Ar¹, S(=O)₂Ar¹, -CR²=CR²(Ar¹), OSO₂R², a straight-chain alkyl, alkoxy or thioalkoxy group having 1 to 40 C atoms or a branched or cyclic alkyl, alkoxy or thioalkoxy group having 3 to 40 C atoms,which may in each case be substituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems; two or more substituents R, R¹ or R and R¹ may also form a mono- or polycyclic, aliphatic, aromatic or heteroaromatic ring system with one another;
Ar¹ is on each occurrence, identically or differently, an aromatic or heteroaromatic ring system having 5 to 60 aromatic ring atoms, which may be substituted by one or more radicals R²;
R² is on each occurrence, identically or differently, H or an aliphatic, aromatic and/or heteroaromatic hydrocarbon radical having 1 to 20 C atoms; or an aromatic or heteroaromatic ring system having 5 to 60 aromatic ring atoms, which may be substituted by one or more radicals R; two or more substituents R² may also form a mono- or polycyclic, aliphatic, aromatic or heteroaromatic ring system with one another;
n is on each occurrence, identically or differently, 0, 1, 2, 3 or 4;
p is on each occurrence, identically or differently, 0, 1, 2, 3 or 4; and
q is 1, 2, 3, 4 or 5.

3. Formulation according to Claim 1, **characterised in that** the triazine compound is a compound of the formula (7) or (8), 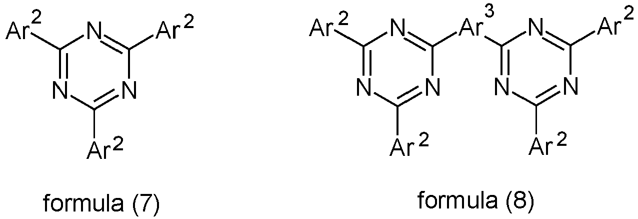 where the following applies to the other symbols used:
Ar¹ is on each occurrence, identically or differently, an aromatic or heteroaromatic ring system having 5 to 40 aromatic ring atoms, which may be substituted by one or more radicals R²;
Ar² is, identically or differently on each occurrence, a monovalent aromatic or heteroaromatic ring system having 5 to 60 aromatic ring atoms, which may in each case be substituted by one or more radicals R¹;
Ar³ is a divalent aromatic or heteroaromatic ring system having 5 to 60 aromatic ring atoms, which may be substituted by one or more radicals R¹;
R¹ is on each occurrence, identically or differently, H, D, F, Cl, Br, I, CHO, C(=O)Ar¹, P(=O)(Ar¹)₂, S(=O)Ar¹, S(=O)₂Ar¹, CR²=CR²Aᵣ¹, CN, NO₂, Si(R²)₃, B(OR²)₂, B(R²)₂, B(N(R²)₂)₂, OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, which may in each case be substituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a combination of these systems; two or more adjacent substituents R¹ may also form a mono- or polycyclic, aliphatic, aromatic or heteroaromatic ring system with one another, and
R² is on each occurrence, identically or differently, H, D, CN or an aliphatic, aromatic and/or heteroaromatic hydrocarbon radical having 1 to 20 C atoms, in which, in addition, H atoms may be replaced by F; two or more adjacent substituents R² may also form a mono- or polycyclic, aliphatic, aromatic or heteroaromatic ring system with one another.

4. Formulation according to one or more of Claims 1 to 3, **characterised in that** the triplet emitter compound is a compound of the formulae (23) to (26), 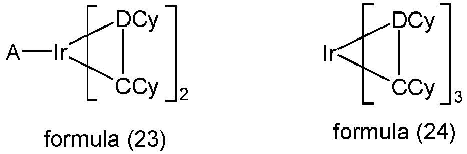 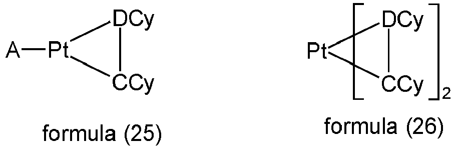 where R¹ has the same meaning as described in Claim 2, and the following applies to the other symbols used:
DCy is, identically or differently on each occurrence, a cyclic group which contains at least one donor atom, preferably nitrogen, carbon in the form of a carbene or phosphorus, via which the cyclic group is bonded to the metal, and which may in turn carry one or more substituents R¹; the groups DCy and CCy are bonded to one another via a covalent bond;
CCy is, identically or differently on each occurrence, a cyclic group which contains a carbon atom via which the cyclic group is bonded to the metal and which may in turn carry one or more substituents R¹; and
A is, identically or differently on each occurrence, a monoanionic, bidentate chelating ligand, preferably a diketonate ligand.

5. Use of a formulation according to one or more of Claims 1 to 4 for the production of an organic electroluminescent device.

6. Organic electroluminescent device comprising cathode, anode and at least one electroluminescent layer, **characterised in that** the electroluminescent layer comprises a mixture comprising a carbazole compound, an electron-transport compound and a triplet emitter compound, where the electron-transport unit comprises a triazine compound and where the carbazole compound contains at least two carbazole groups which are connected to one another via their N atoms.

7. Organic electroluminescent device according to Claim 6, **characterised in that** the electroluminescent layer has been obtained from a formulation according to one of Claims 1 to 5.

8. Organic electroluminescent device according to Claim 6 or 7, **characterised in that** no electron-injection layer is present in the device.

9. Organic electroluminescent device according to one or more of Claims 6 to 8, **characterised in that** the electroluminescent layer has been applied from solution.

## Revendications

1. Formulation comprenant un composé de carbazole, un composé de transport d'électrons, un composé émetteur de triplets et au moins un solvant, dans laquelle le composé de transport d'électrons comprend un composé de triazine et dans laquelle le composé de carbazole contient au moins deux groupes de carbazole qui sont connectés l'un à l'autre ou les uns aux autres via leurs atomes de N.

2. Formulation selon la revendication 1, **caractérisée en ce que** le composé de carbazole est un composé de la formule (1) 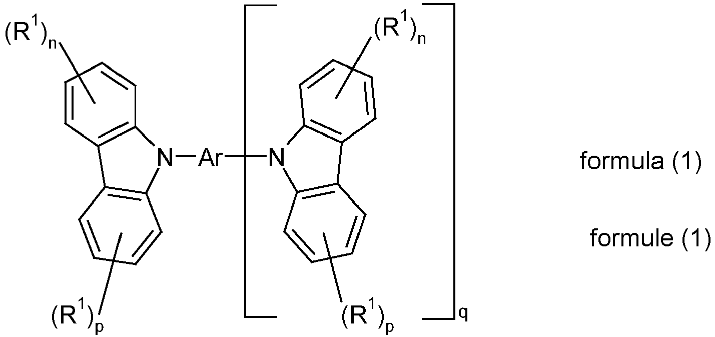 dans laquelle ce qui suit s'applique aux symboles et aux indices qui sont utilisés :
Ar est pour chaque occurrence un système de cycle aromatique ou hétéroaromatique qui comporte de 5 à 60 atomes de cycle aromatique, lequel peut être substitué par un radical ou par plusieurs radicaux R ;
R, R¹ sont pour chaque occurrence, de manière identique ou différente, H, D, F, Cl, Br, I, N(Ar¹)₂, CN, NO₂, Si(R ²)₃ , B(OR²)₂, C(=O)Ar¹, P(=O)(Ar¹)₂, S(=O)Ar¹, S(=O)₂Ar¹, -CR²=CR²(Ar¹), OSO₂R², un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 60 atomes de cycle aromatique, lequel peut être substitué par un radical ou par plusieurs radicaux R², ou une combinaison de ces systèmes ; deux substituants R, R¹ ou R et R¹ ou plus peuvent également former un système de cycle aliphatique, aromatique ou hétéroaromatique mono- ou polycyclique l'un avec l'autre ou les uns avec les autres ;
Ar¹ est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R² ;
R² est pour chaque occurrence, de manière identique ou différente, H ou un radical hydrocarbone aliphatique, aromatique et/ou hétéroaromatique qui comporte de 1 à 20 atome(s) de C ; ou un système de cycle aromatique ou hétéroaromatique qui comporte de 5 à 60 atomes de cycle aromatique, lequel peut être substitué par un radical ou par plusieurs radicaux R ; deux substituants R² ou plus peuvent également former un système de cycle aliphatique, aromatique ou hétéroaromatique mono- ou polycyclique l'un avec l'autre ou les uns avec les autres ;
n est pour chaque occurrence, de manière identique ou différente, 0, 1, 2, 3 ou 4 ;
p est pour chaque occurrence, de manière identique ou différente, 0, 1, 2, 3 ou 4 ; et
q est 1, 2, 3, 4 ou 5.

3. Formulation selon la revendication 1, **caractérisée en ce que** le composé de triazine est un composé de la formule (7) ou (8), 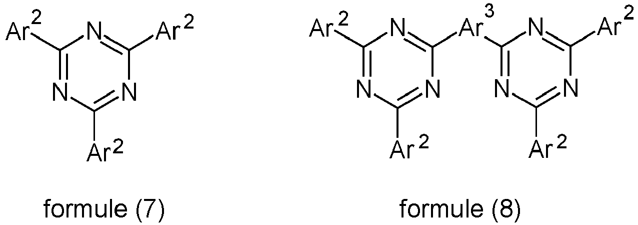 dans lesquelles ce qui suit s'applique aux autres symboles qui sont utilisés :
Ar¹ est pour chaque occurrence, de manière identique ou différente, un système de cycle aromatique ou hétéroaromatique qui comporte de 5 à 40 atomes de cycle aromatique, lequel peut être substitué par un radical ou par plusieurs radicaux R² ;
Ar² est, de manière identique ou différente pour chaque occurrence, un système de cycle aromatique ou hétéroaromatique monovalent qui comporte de 5 à 60 atomes de cycle aromatique, lequel peut dans chaque cas être substitué par un radical ou par plusieurs radicaux R¹ ;
Ar³ est un système de cycle aromatique ou hétéroaromatique divalent qui comporte de 5 à 60 atomes de cycle aromatique, lequel peut être substitué par un radical ou par plusieurs radicaux R¹;
R¹ est pour chaque occurrence, de manière identique ou différente, H, D, F, Cl, Br, I, CHO, C(=O)Ar¹, P(=O)(Ar¹)₂, S(=O)Ar¹, S(=O)₂Ar¹, CR²=CR²Aᵣ¹, CN, NO₂, Si(R²)₃, B(OR²)₂, B(R²)₂, B(N(R²)₂)₂, OSO₂R², un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 60 atomes de cycle aromatique, lequel peut être substitué par un radical ou par plusieurs radicaux R², ou une combinaison de ces systèmes ; deux substituants R¹ adjacents ou plus peuvent également former un système de cycle aliphatique, aromatique ou hétéroaromatique mono- ou polycyclique l'un avec l'autre ou les uns avec les autres, et
R² est pour chaque occurrence, de manière identique ou différente, H, D, CN ou un radical hydrocarbone aliphatique, aromatique et/ou hétéroaromatique qui comporte de 1 à 20 atome(s) de C, où, en outre, des atomes de H peuvent être remplacés par F ; deux substituants R² adjacents ou plus peuvent également former un système de cycle aliphatique, aromatique ou hétéroaromatique mono- ou polycyclique l'un avec l'autre ou les uns avec les autres.

4. Formulation selon une ou plusieurs des revendications 1 à 3, **caractérisée en ce que** le composé émetteur de triplets est un composé des formules (23) à (26), 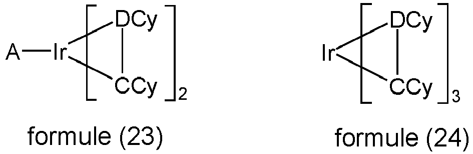 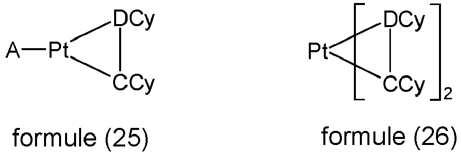 dans lesquelles R¹ présente la même signification qu'il a été décrit selon la revendication 2, et ce qui suit s'applique aux autres symboles qui sont utilisés :
DCy est, de manière identique ou différente pour chaque occurrence, un groupe cyclique qui contient au moins un atome donneur, de préférence un atome d'azote, de carbone sous la forme d'un carbène ou de phosphore, via lequel le groupe cyclique est lié au métal, et lequel peut à son tour être porteur d'un ou de plusieurs substituant(s) R¹ ; les groupes DCy et CCy sont liés l'un à l'autre via une liaison covalente ;
CCy est, de manière identique ou différente pour chaque occurrence, un groupe cyclique qui contient un atome de carbone via lequel le groupe cyclique est lié au métal et lequel peut à son tour être porteur d'un ou de plusieurs substituant(s) R¹ ; et
A est, de manière identique ou différente pour chaque occurrence, un ligand chélatant bidenté monoanionique, de préférence un ligand dicétonate.

5. Utilisation d'une formulation selon une ou plusieurs des revendications 1 à 4 pour la fabrication d'un dispositif électroluminescent organique.

6. Dispositif électroluminescent organique comprenant une cathode, une anode et au moins une couche électroluminescente, **caractérisé en ce que** la couche électroluminescente comprend un mélange qui comprend un composé de carbazole, un composé de transport d'électrons et un composé émetteur de triplets, dans lequel l'unité de transport d'électrons comprend un composé de triazine et dans lequel le composé de carbazole contient au moins deux groupes carbazole qui sont connectés l'un à l'autre ou les uns aux autres via leurs atomes de N.

7. Dispositif électroluminescent organique selon la revendication 6, **caractérisé en ce que** la couche électroluminescente a été obtenue à partir d'une formulation selon une ou plusieurs des revendications 1 à 5.

8. Dispositif électroluminescent organique selon la revendication 6 ou 7, **caractérisé en ce qu'**aucune couche d'injection d'électrons n'est présente dans le dispositif.

9. Dispositif électroluminescent organique selon une ou plusieurs des revendications 6 à 8, **caractérisé en ce que** la couche électroluminescente a été appliquée à partir d'une solution.
